# EUROPEAN PATENT SPECIFICATION

(11) **EP 3 092 995 B1**
(45) Date of publication and mention of the grant of the patent: **30.01.2019**
(21) Application number: 14877653.7
(22) Date of filing: 09.10.2014
(51) Int. Cl.: B29C 65/08, A61F 13/15, A61F 13/49, B29C 65/78, B29L 31/48

(54) **ULTRASONIC WELDING APPARATUS AND ULTRASONIC WELDING METHOD OF SHEET-LIKE MEMBER ASSOCIATED WITH ABSORBENT ARTICLE**
ULTRASCHALLSCHWEISSVORRICHTUNG UND ULTRASCHALLSCHWEISSVERFAHREN FÜR BAHNENFÖRMIGES ELEMENT IM ZUSAMMENHANG MIT SAUGFÄHIGEM ARTIKEL
DISPOSITIF DE SOUDAGE ULTRASONORE ET PROCÉDÉ DE SOUDAGE ULTRASONORE D'ÉLÉMENT EN FORME DE FEUILLE ASSOCIÉ À UN ARTICLE ABSORBANT

(30) Priority: 10.01.2014 JP 2014002986
(43) Date of publication of application: 16.11.2016
(73) Proprietor: Unicharm Corporation, Ehime 799-0111 (JP)
(72) Inventor: YAMAMOTO, Hiroki, Kanonji-shi Kagawa 769-1602 (JP); MATSUMOTO, Yoshihiko, Kanonji-shi Kagawa 769-1602 (JP)
(74) Representative: Dolleymores
(86) International application number: PCT/JP2014/077104
(87) International publication number: WO 2015/104880

(56) References cited:
- WO-A1-2012/042842
- WO-A1-2013/027390
- WO-A1-2013/027390
- WO-A1-2013/141022
- CN-A- 1 179 128
- JP-A- H10 291 082
- JP-A- H10 513 128
- JP-A- S62 282 914
- JP-A- 2000 015 701
- US-A- 5 643 396
- US-A- 5 667 608
- US-A1- 2013 167 629

## Description

### Technical Field

The invention relates to an ultrasonic welding apparatus and an ultrasonic welding method of welding a sheet-like member using ultrasonic vibration, the sheet-like member being associated with an absorbent article such as a disposable diaper.

### Background Art

In a conventional production line of an absorbent article such as a disposable diaper, a sheet-like member 1a, which is composed of a plurality of stacked continuous sheets (e.g. nonwoven fabric), is welded so that these continuous sheets are joined. As an example of an apparatus 120 which perform such a welding process, [Patent Literature 1] discloses an ultrasonic welding apparatus 120. The apparatus 120 generates friction heat by applying ultrasonic energy to the sheet-like member 1a, and thereby welds the sheet-like member 1a.

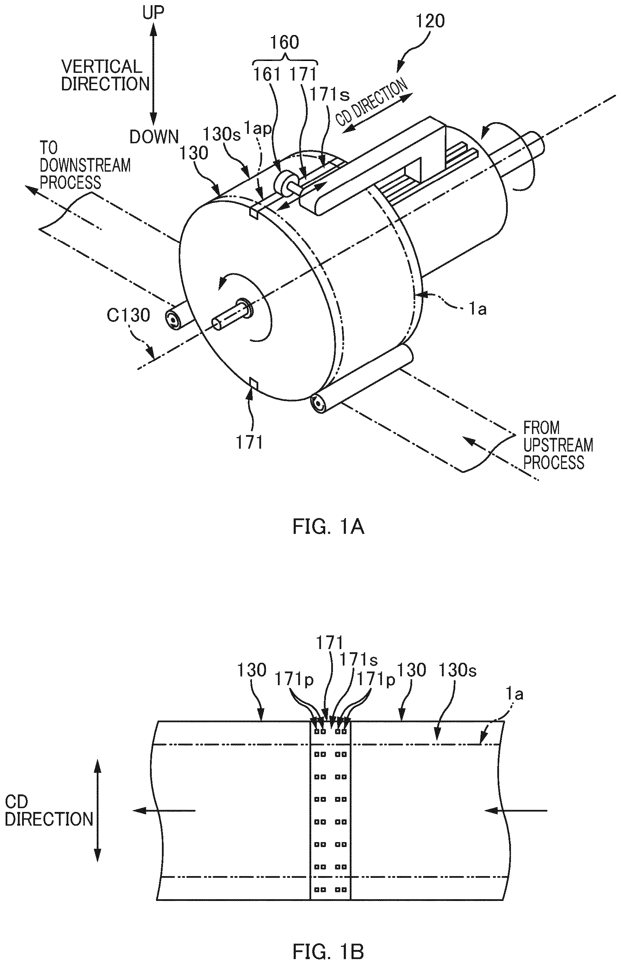

FIG. 1A is a schematic perspective view of the apparatus 120. The apparatus 120 includes a rotating drum 130 which rotates about a central axis C130. A sheet-like member 1a to be welded (see double-dotted chain lines in FIG. 1A) is wound around the outer circumferential face 130s of the rotating drum 130, and the rotating drum 130 rotates about the central axis C130. Accordingly, the sheet-like member 1a is substantially integrated with the outer circumferential face 130s of the rotating drum 130 and is being transported.

The apparatus 120 also includes an ultrasonic processing unit 160, and the unit 160 rotates about the central axis C130 in an integrated manner with the rotating drum 130. During the rotation, the unit 160 performs ultrasonic welding process to the sheet-like member 1a.

That is, the ultrasonic processing unit 160 includes a rail-like anvil 171 and a roller-like horn 161 on the outer circumferential face 130s of the rotating drum 130. The anvil 171 extends in the CD direction, which is orthogonal to the transport direction. The horn 161 vibrates ultrasonically and is provided outside with respect to the anvil 171 in the rotation-radius direction of the rotating drum 130. During a period in which the horn 161 and the anvil 171 both are rotating about the central axis C130 together with the rotating drum 130 and the sheet-like member 1a, the roller-like horn 161 rolls on the anvil 171 along the CD direction and reciprocates in the CD direction. At this time, the horn 161 applies ultrasonic energy to a part 1ap of the sheet-like member 1a which is placed on the anvil 171, and thereby welds the part 1ap.

### Citation List

### [Patent Literature].

[Patent Literature 1] Japanese Unexamined Patent Application Publication (Translation of PCT Application) No. 10-513128. US 5667608 A and EP0807013 A1 are patent family members.

WO 2012/042842 A1 provides an apparatus and a method for ultrasonic processing a fibrous web, wherein a first mechanical element is defined by one of an ultrasonic horn and an anvil and a second mechanical element is defined by the other of the ultrasonic horn and the anvil, the mechanical elements being moved forward or backward in a direction crossing the machine direction so as to pass transversely across the fibrous web.

The second mechanical element repetitively moves back-and-forth in synchronization with the back-and-forth movement of the first mechanical element in the same direction as the direction in which the first mechanical element moves back-and-forth. The first and second mechanical elements are adapted to cooperate with each other to subject the fibrous web to ultrasonic processing during one of the forward and backward movements, and at least one of the first and second mechanical elements is adapted to be spaced from the fibrous web in order to stop the ultrasonic processing during the other of the forward and backward movements.

CN 1179128 A, US 5643396 A, WO 2013/027390 A1 and US 2013/167629 A1 also relate to ultrasonic processing systems for webs.

### Summary of Invention

### Technical Problem

However, [Patent Literature 1] does not disclose the amount of ultrasonic energy applied in a forward path and a return path for reciprocation.

Here, it is supposed that a welding process is performed by applying the same amount of ultrasonic energy in both of the forward path and the return path. In this case, a welded part (not shown) is formed during the movement in the forward path, and also, a welded part (not shown) is formed during the movement in the return path. The apparatus is designed to form these welded parts, in both of the forward path and the return path, at the same position of the sheet-like member 1a. That is, in the light of the apparatus design, these welded parts are formed completely coinciding.

But, in actual use, it is possible that the positions where the welded parts are formed in the forward path and the return path do not coincide because the sheet-like member 1a, which is wound around the outer circumferential face 130s of the rotating drum 130, is slightly shifted. This worsens the appearance of the welded parts.

FIG. 1B is for describing the problem in more detail, and is a schematic diagram of the outer circumferential face 130s of the rotating drum 130 which is developed view on a plane. Generally, the anvil 171 has a flat, roll surface 171s on which a roller-like horn 161 moves rolling, and on the roll surface 171s, a plurality of protrusions 171p, 171p... are provided. Accordingly, in the ultrasonic welding process, a plurality of depressions (not shown) are formed as a welded part on the sheet-like member 1a, and the depressions are in a pattern corresponding to the plurality of protrusions 171p, 171p.... But, if the sheet-like member 1a is slightly shifted between the forward path and the return path as mentioned above, the depressions, which should be formed in the forward path and the return path so as to coincide, are also formed being shifted. Consequently, the welded parts formed in the return path do not completely superpose the welded parts formed in the forward path, and the appearance of the welded parts becomes bad. This decreases the commodity value of the absorbent article.

In this regard, in either one of the forward path and the return path, when the anvil 171 or the horn 161 is separated from the sheet-like member 1a, the welded part is formed in either one of the forward path or the return path. This enables the positional variation of the welded parts to be inconspicuous.

But, a single ultrasonic processing unit 160 usually performs tens or one hundred tens times of welding processes per minute. The reciprocation of the horn 161 in the CD direction is performed in hundreds millisecond. Accordingly, the abovementioned separating operation is required to be performed within a part of a period of hundreds milliseconds. But, it is actually difficult to perform the separating operation in such small amounts of time.

The invention has been made in view of the above conventional problems, and an advantage thereof is, when ultrasonic welding process is performed in a predetermined section in the CD direction, concerning the positional variation between a welded part formed in the predetermined section of a forward path and a welded part formed in the predetermined section of a return path, to make the positional variation inconspicuous without separating an anvil and a horn from a sheet-like member in the predetermined section in both of the forward path and the return path.

### Solution to Problem

The present invention provides the ultrasonic welding apparatus of independent claim 1 and the ultrasonic welding method of independent claim 7. The dependent claims specify preferred but optional features.

Other features of this invention will become apparent from the description in this specification and the attached drawings.

### Advantageous Effects of Invention

According to the invention, when ultrasonic welding process is performed in a predetermined section in the CD direction, concerning the positional variation between a welded part formed in the predetermined section of a forward path and a welded part formed in the predetermined section of a return path, the positional variation can be inconspicuous without separating an anvil and a horn from a sheet-like member in the predetermined section in both of the forward path and the return path.

### Brief Description of Drawings

FIG. 1A is a schematic perspective view of a conventional ultrasonic welding apparatus 120. FIG. 1B is a schematic diagram of the outer circumferential face 130s of the rotating drum 130 which is developed view on a plane, and is for explaining a problem of the positional variation of welded parts.
FIG. 2A is a schematic perspective view of a substrate 1a of a diaper 1 which has not two-folded yet, and FIG. 2B is a schematic perspective view of the two-folded substrate 1a immediately before being transported to an ultrasonic welding apparatus 20 according to the reference example.
FIG. 3 is a schematic perspective view of the ultrasonic welding apparatus 20 of the reference example as viewed obliquely from front above.
FIG. 4 is a schematic side view along arrows IV-IV in FIG. 3.
FIG. 5 is a schematic front view along arrows V-V in FIG. 3.
FIG. 6 is a partially cutaway, schematic side view of the ultrasonic welding apparatus 20 in which the substrate 1a and a rotating drum 30 are removed from FIG. 4.
FIG. 7A is a schematic perspective view of a support unit 73 as viewed obliquely from front and outside in a rotation-radius direction Dr30. FIG. 7B is a schematic perspective view of the support unit 73 as viewed obliquely from back and outside in the rotation-radius direction Dr30.
FIG. 8 is a schematic front view of the ultrasonic welding apparatus 20 and shows the ranges in a rotation direction Dc30 to which a forward movement and a backward movement are respectively allocated, the forward movement being movement in a forward path, the backward movement being movement in a return path.
FIG. 9 is a schematic perspective view of an anvil roller 71.
FIG. 10 is a schematic diagram of the outer circumferential face 30s of the rotating drum 30 and is for explaining a crossing section A1a.
FIG. 11 is a schematic diagram of an ultrasonic processing unit 60a according to the second modified reference example as viewed in the rotation direction Dc30 of the rotating drum 30.
FIG. 12 is a schematic front view of the ultrasonic welding apparatus 20, and is for explaining a cam curve according to the third modified reference example.
FIG. 13 is a schematic perspective view of an ultrasonic welding apparatus 20b according to the first embodiment as viewed obliquely from front above.
FIG. 14 is a schematic side view along arrows XIV-XIV in FIG. 13.
FIG. 15 is a schematic front view along arrows XV-XV in FIG. 13.
FIG. 16 is a schematic side view of the apparatus 20b, in which the substrate 1a and the rotating drum 30 are removed from FIG. 14.
FIG. 17A is a schematic perspective view of an ultrasonic processing unit 60b according to the first embodiment as viewed obliquely from front and outside in the rotation-radius direction Dr30, and FIG. 17B is a schematic perspective view of the unit 60b as viewed obliquely from back and outside.
FIG. 18 is a schematic perspective view of an ultrasonic processing unit 60c showing another example of the positional relation of a horn 61b and the anvil roller 71 according to the first embodiment. Description of Reference Examples and Embodiments

At least the following matters will be made clear by the description in the present specification and the accompanying drawings.

An ultrasonic welding apparatus that performs an ultrasonic welding process to a sheet-like member,
the sheet-like member being associated with an absorbent article,
the sheet-like member being transported while being wound onto an outer circumferential face of a rotating member,
the rotating member rotating about its central axis,
the apparatus, including:
the rotating member;
an ultrasonic processing unit
   that rotates about the central axis together with the rotating member, and
   that includes as ultrasonic processing members
      a horn that vibrates ultrasonically and
      an anvil between which and the horn the sheet-like member is sandwiched; and
an ultrasonic-energy regulating unit,
   the horn and the anvil being guided so as to reciprocate in a CD direction,
      the CD direction intersecting a transport direction of the sheet-like member,
   the horn and the anvil reciprocating in the CD direction with respect to a part of the sheet-like member,
      the part being wound around the rotating member,
   both of the horn and the anvil being in contact with the sheet-like member in a predetermined section during movement in both of a forward path and a return path of the reciprocation,
      the predetermined section being included in the outer circumferential face of the rotating member and extending in the CD direction,
   the ultrasonic-energy regulating unit making
      an amount (J/m) of applied ultrasonic energy per unit length in the CD direction of the predetermined section of the forward path
         different from
         an amount (J/m) of applied ultrasonic energy per unit length in the CD direction of the predetermined section of the return path.

With such an ultrasonic welding apparatus of a sheet-like member associated with an absorbent article, the amount (J/m) of ultrasonic energy applied in the abovementioned predetermined section is different between the forward path and the return path of the ultrasonic processing member. Accordingly, in either one of the forward path and the return path, the ultrasonic welding process can be performed to a considerable extent in the predetermined section. On the other hand, in the other path, the extent of the ultrasonic welding process can be reduced in the predetermined section. Therefore, even if there is a positional variation between a welded part formed in the forward path and a welded part formed in the return path, the positional variation can be inconspicuous.

In the predetermined section, during movement in both of the forward path and the return path, the horn and the anvil are in contact with the sheet-like member. Accordingly, in the predetermined section, it is unnecessary to separate the anvil and the horn from the sheet-like member. Consequently, the apparatus can be easily realized.

In such an ultrasonic welding apparatus of a sheet-like member associated with an absorbent article, it is preferable that
the ultrasonic-energy regulating unit changes an amount of the ultrasonic energy (J/m) that is applied per unit length, by changing an amplitude of ultrasonic vibration of the horn.

With such an ultrasonic welding apparatus of a sheet-like member associated with an absorbent article, the amount (J/m) of ultrasonic energy is changed by changing the amplitude of the ultrasonic vibration. This makes it possible to change easily the amount (J/m) of ultrasonic energy with high response.

In such an ultrasonic welding apparatus of a sheet-like member associated with an absorbent article, it is preferable that
the ultrasonic-energy regulating unit changes an amount of the ultrasonic energy (J/m) that is applied per unit length, by changing a magnitude of sandwiching force at which the sheet-like member is sandwiched between the horn and the anvil.

With such an ultrasonic welding apparatus of a sheet-like member associated with an absorbent article, the amount of the ultrasonic energy (J/m) is changed by changing the magnitude of the sandwiching force. This makes it possible to easily and reliably change the amount of the ultrasonic energy (J/m).

In such an ultrasonic welding apparatus of a sheet-like member associated with an absorbent article, it is preferable that
the ultrasonic-energy regulating unit changes an amount of the ultrasonic energy (J/m) that is applied per unit length, by changing a size of a space between the horn and the anvil.

With such an ultrasonic welding apparatus of a sheet-like member associated with an absorbent article, the amount of the ultrasonic energy (J/m) is changed by changing the size of the space between the horn and the anvil. This makes it possible to easily and reliably change the amount of the ultrasonic energy (J/m).

In such an ultrasonic welding apparatus of a sheet-like member associated with an absorbent article, it is preferable that
the ultrasonic-energy regulating unit changes an amount of the ultrasonic energy (J/m) that is applied per unit length, by changing a travel speed value at which the ultrasonic processing member moves in the CD direction.

With such an ultrasonic welding apparatus of a sheet-like member associated with an absorbent article, the amount of the ultrasonic energy (J/m) is changed by changing the travel speed value at which the ultrasonic processing member moves in the CD direction. This makes it possible to reliably change the amount of the ultrasonic energy (J/m).

In such an ultrasonic welding apparatus of a sheet-like member associated with an absorbent article, both of the horn and the anvil reciprocate in the CD direction while the sheet-like member is sandwiched between the horn and the anvil.

With such an ultrasonic welding apparatus of a sheet-like member associated with an absorbent article, both of the horn and the anvil move in the CD direction relative to the sheet-like member. Though weld residue will adhere to and is accumulated on at least horn as a result of the ultrasonic welding process, it is possible to wipe successively weld residue by contact with the sheet-like member during the reciprocation in the CD direction. This makes it possible to effectively prevent accumulation of weld residue on the horn.

In such an ultrasonic welding apparatus of a sheet-like member associated with an absorbent article, it is preferable that
in at least either one of the horn and the anvil, a plurality of protrusions are provided so that welded parts are formed in a pattern in the sheet-like member.

With such an ultrasonic welding apparatus of a sheet-like member associated with an absorbent article, a plurality of protrusions form the welded parts in a pattern. This can improve the design of the welded parts.

If the welded parts are in a pattern, when there is a positional variation between the welded part formed in the forward path and the welded part formed in the return path, the positional variation is more likely to be noticed. In this case, it is therefore possible to effectively achieve working effect of the invention, that is, making the positional variation inconspicuous.

In such an ultrasonic welding apparatus of a sheet-like member associated with an absorbent article,
in both of the forward path and the return path, the horn vibrates ultrasonically when the ultrasonic processing member is passing the predetermined section.

With such an ultrasonic welding apparatus of a sheet-like member associated with an absorbent article, when the ultrasonic processing member is passing the abovementioned predetermined section, the horn vibrates ultrasonically in both of the forward path and the return path of reciprocation of the ultrasonic processing member. That is, when the ultrasonic processing member is passing the predetermined section, the horn is vibrating in a direction in which the sheet-like member is sandwiched. This can reduce kinetic frictional resistance which may generate between the ultrasonic processing member and the sheet-like member when the ultrasonic processing member is passing the predetermined section. Accordingly, the ultrasonic processing member can reciprocate in the CD direction smoothly.

Further, an ultrasonic welding method in which a sheet-like member associated with an absorbent article is subject to an ultrasonic welding process,
the sheet-like member being transported on an outer circumferential face of a rotating member, the rotating member rotating about its central axis,
the method including:
rotating an ultrasonic processing unit about the central axis together with the rotating member,
   the ultrasonic processing unit including as ultrasonic processing members
   a horn and
   an anvil facing the horn;
causing the horn to vibrate ultrasonically;
causing the horn and the anvil to reciprocate in a CD direction with respect to a part of the sheet-like member, the CD direction intersecting a transport direction of the sheet-like member,
   the part being wound around the rotating member,
   the reciprocating being performed while the sheet-like member is sandwiched between the horn and the anvil;
   the horn and the anvil being in contact with the sheet-like member in a predetermined section during movement in both of a forward path and a return path of the reciprocation,
   the predetermined section being included in the outer circumferential face of the rotating member and extending in the CD direction,
an amount (J/m) of applied ultrasonic energy per unit length in the CD direction of the predetermined section of the forward path being different from
an amount (J/m) of applied ultrasonic energy per unit length in the CD direction of the predetermined section of the return path.

With such an ultrasonic welding method for a sheet-like member associated with an absorbent article, the amount (J/m) of ultrasonic energy applied in the abovementioned predetermined section is different between the forward path and the return path of the ultrasonic processing member. Accordingly, in either one of the forward path and the return path, the ultrasonic welding process can be performed to a considerable extent in the predetermined section. On the other hand, in the other path, the extent of the ultrasonic welding process can be reduced in the predetermined section. Therefore, even if there is a positional variation between a welded part formed in the forward path and a welded part formed in the return path, the positional variation can be inconspicuous.

In the predetermined section, during movement in both of the forward path and the return path, the horn and the anvil are in contact with the sheet-like member. Accordingly, in the predetermined section, it is unnecessary to separate the anvil and the horn from the sheet-like member. Consequently, the method can be easily realized.

### === Reference example ===

An ultrasonic welding apparatus 20 according to the invention is an apparatus which forms a welded part 14 in a continuous sheet-like member 1a at intervals in the transport direction (e.g. a predetermined pitch PI), the sheet-like member 1a being transported in production line. In this reference example, the sheet-like member 1a is exemplified by the substrate 1a of pull-on disposable diapers 1.

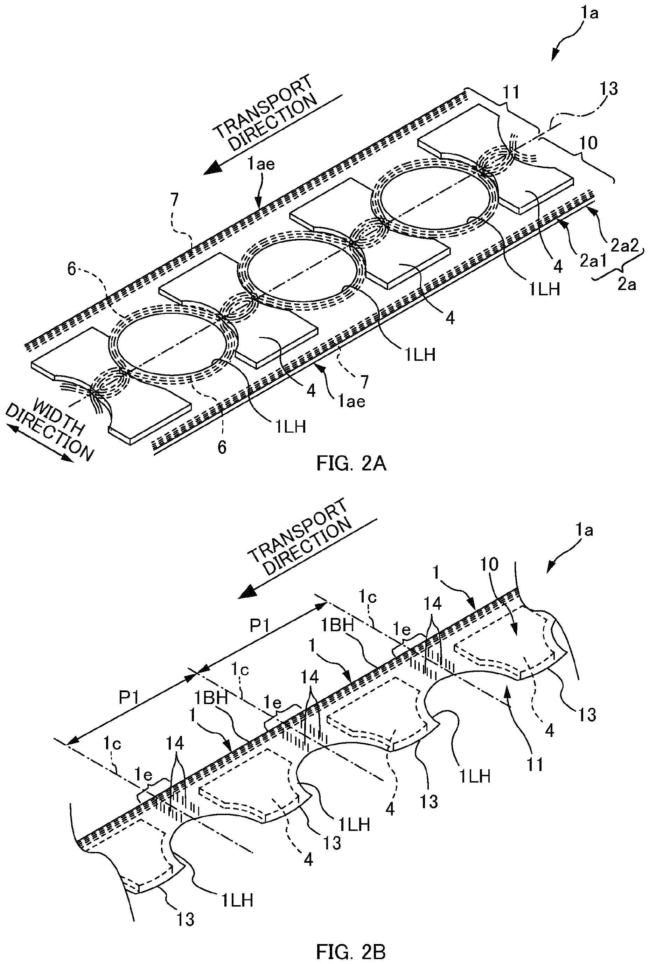

FIGS. 2A and 2B are schematic perspective views and illustrate how the substrate 1a of the diaper 1 is transported to the ultrasonic welding apparatus 20. FIG. 2A shows the substrate 1a of the diaper 1 which has not two-folded yet, and FIG. 2B shows the two-folded substrate 1a immediately before being transported to the ultrasonic welding apparatus 20.

The substrate 1a of the diaper 1 includes a continuous sheet 2a which continues in the transport direction. At the time point shown in FIG. 2A, the substrate 1a is in a state in which, on a surface of the continuous sheet 2a which is to face wearer's skin, absorbent main bodies 4, 4... are placed on the substrate 1a with spacing of a product pitch P1 in the transport direction and joined to the substrate 1a with adhesive or the like.

In the substrate 1a at this time, leg openings 1LH are formed at a position between absorbent main bodies 4 and 4 which are adjacent to each other in the transport direction. Leg-circumference elastic members 6, which provide stretchability to the leg openings 1LH, are attached along the leg opening 1LH. In addition, waist-circumference elastic members 7, which provide stretchability to the waist opening 1BH, are attached along edges 1ae and 1ae which are to be the waist opening 1BH.

The continuous sheet 2a is composed of a sheet 2a having a two layer structure, for example. That is, the continuous sheet 2a includes: a continuous sheet 2a1 (hereinafter referred to as an inner-layer sheet 2a1) which faces the skin side of a wearer to serve as an inner layer; and a continuous sheet 2a2 (hereinafter referred to as an outer-layer sheet 2a2) which faces the non-skin side of a wearer to serve as an outer layer. The inner-layer sheet 2a1 and the outer-layer sheet 2a2 are stacked in the thickness direction and are joined by means such as adhesion or welding. It is to be noted that an example of raw material of these continuous sheets 2a1 and 2a2 includes nonwoven fabric, woven fabric or a film made of a thermally weldable material such as thermoplastic resin. But, the invention is not limited thereto as long as it is a material capable of being ultrasonically welded, that is, a material capable of being melted and joined by heat which is generated by friction due to application of ultrasonic energy.

The absorbent main body 4 is a component which absorbs excretion liquid, and the main body thereof is a body formed by shaping liquid absorbent fiber (e.g. pulp fiber) or liquid-absorbent particulate matter (e.g. super absorbent polymer) into a predetermined body (e.g. a substantially hourglass shape). Such a shaped body is covered with a liquid-permeable cover sheet (not shown) such as tissue paper or nonwoven fabric. In addition, the shaped body is covered with a liquid-impermeable leak-proof sheet from the non-skin side.

Immediately before being fed into the ultrasonic welding apparatus 20, such a substrate 1a shown in FIG. 2A is two-folded in a crotch part 13, which is the substantially center of the substrate 1a in its width direction. Then, the substrate 1a which has been two-folded as shown in FIG. 2B is transported to the ultrasonic welding apparatus 20. In other words, a part corresponding to the front piece 10 of a diaper 1 and a part corresponding to the back piece 11 are stacked in the up-down direction and are transported to the ultrasonic welding apparatus 20.

However, at this time, the substrate 1a of the diaper 1 is in a state in which the part corresponding to the front piece 10 and the part corresponding to the back piece 11 are stacked but have not been joined yet. Accordingly, the ultrasonic welding apparatus 20 forms the welded part 14 of the substrate 1a by welding the substrate 1a on its part 1e which corresponds to waist-circumferential side-end parts 1e of a diaper 1. Consequently, the front piece 10 and the back piece 11 of the substrate 1a are joined.

Here, the parts 1e which are to be welded, that is, the parts 1e each of which corresponds to waist-circumferential side-end parts 1e of a diaper 1 are placed in the substrate 1a at a product pitch P1 in the transport direction on both sides of each absorbent main body 4. Accordingly, the ultrasonic welding apparatus 20 forms welded parts 14 on both side parts 1e of each absorbent main body 4 in the substrate 1a, at the product pitch P1 in the transport direction. At this time, as shown in FIG. 2B, for each of to-be-welded parts 1e, at least a pair of welded parts 14 and 14 are formed being lined in the transport direction. Then, the substrate 1a in which these welded parts 14 are formed is transported to a downstream process, and in the downstream process, the substrate 1a is subsequently divided at a part 1c, which is between the pair of welded parts 14 and 14. Thereby, a diaper 1 having the waist opening 1BH and the leg openings 1LH is produced.

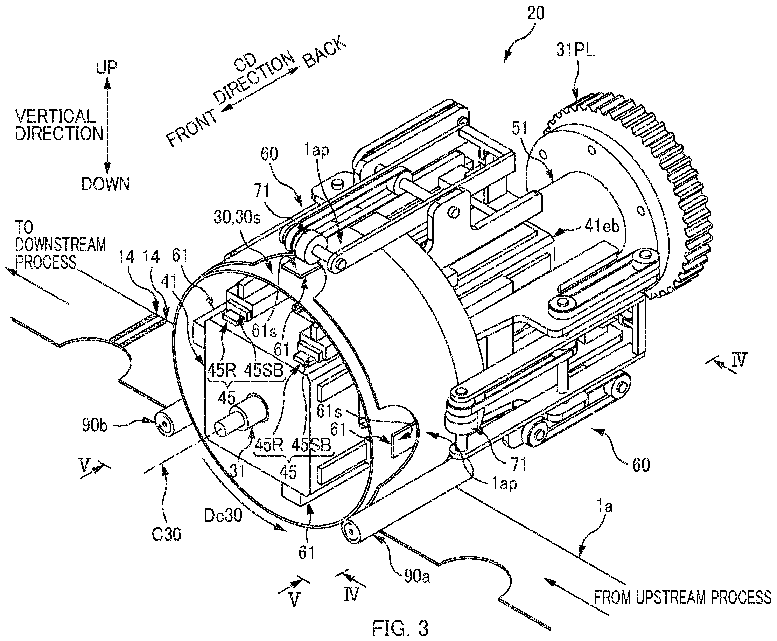

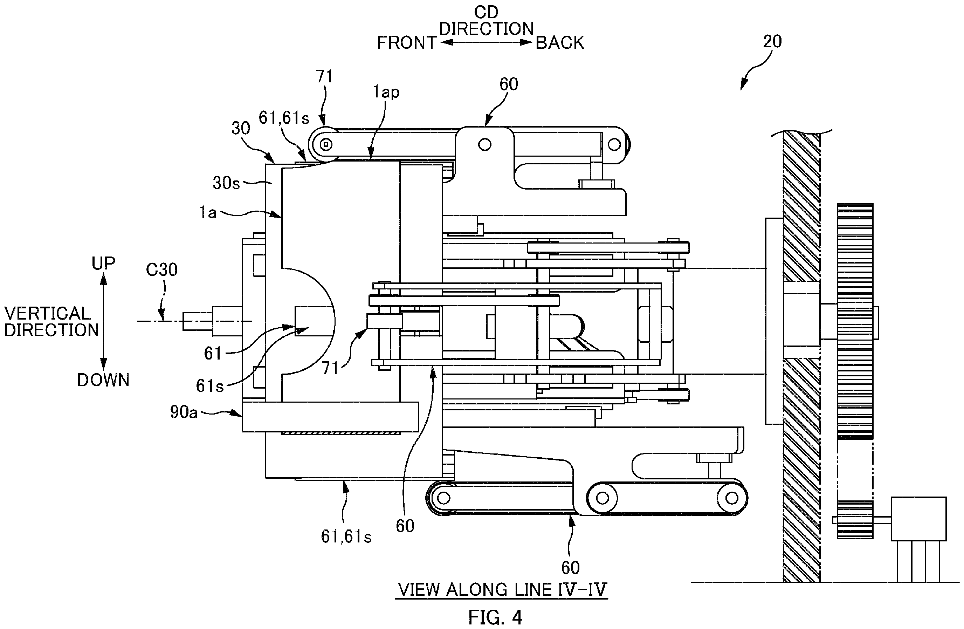

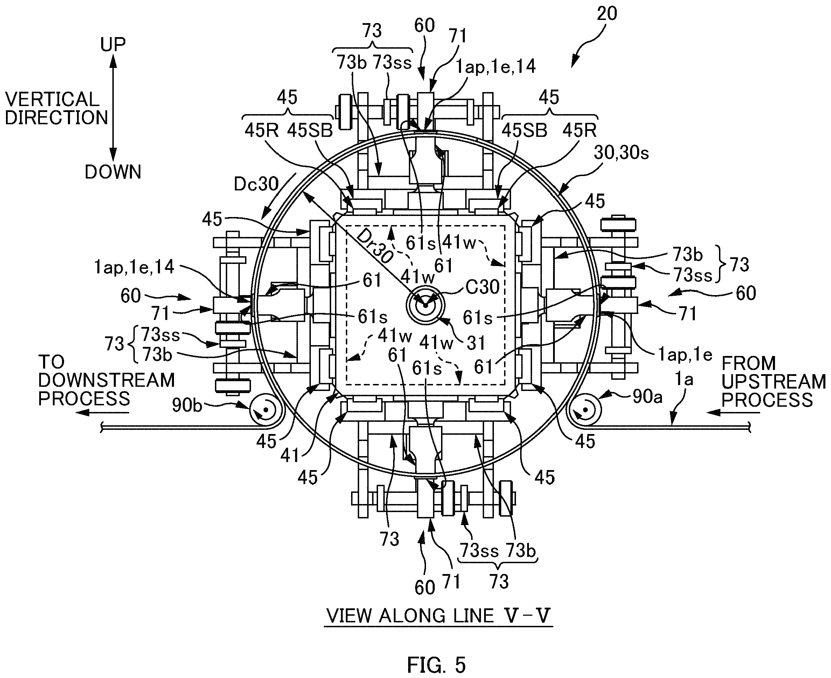

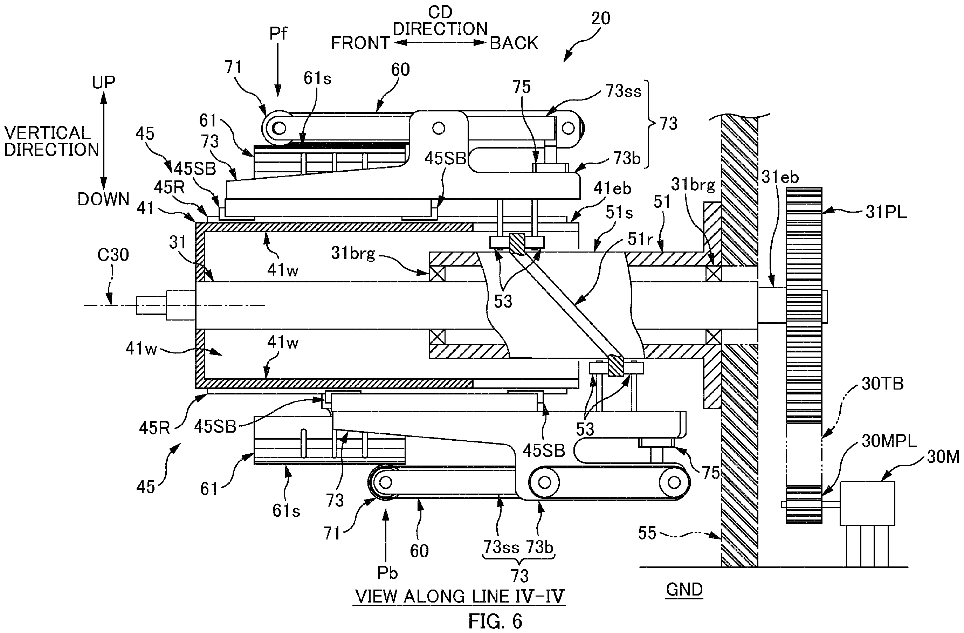

FIG. 3 is a schematic perspective view of the ultrasonic welding apparatus 20 as viewed obliquely from front above. FIG. 4 is a schematic side view along arrows IV-IV in FIG. 3, and FIG. 5 is a schematic front view along arrows V-V in FIG. 3. FIG. 6 is a schematic side view of the ultrasonic welding apparatus 20. In FIG. 4, the substrate 1a and a rotating drum 30 are removed, and some components such as a column 41 are illustrated in a cut-away view.

In the description below, a direction orthogonal to the transport direction of the substrate 1a in the production line is referred to as a "CD direction". In this example, the CD direction is in the horizontal direction. And, a substrate 1a is being transported along a continuing direction in which the substrate 1a continues, and the width direction of the substrate 1a is designed to be parallel to the abovementioned CD direction. The thickness direction of the substrate 1a is orthogonal to both of the continuing direction and the width direction of the substrate 1a.

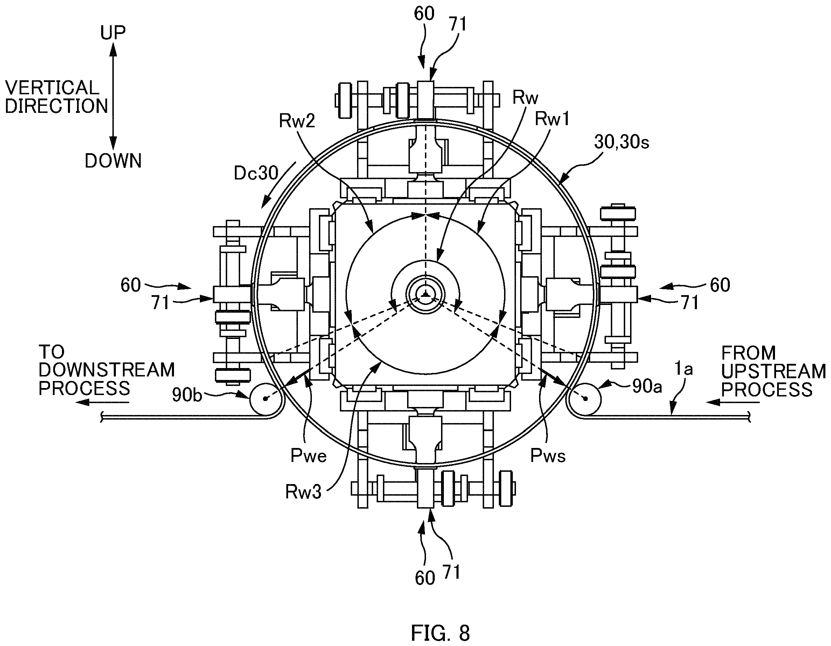

As shown in FIGS. 3 and 5, the ultrasonic welding apparatus 20 includes: the rotating drum 30 (corresponding to the rotating member); a plurality (four in this example) of ultrasonic processing units 60, 60...; and a pair of guide rolls 90a and 90b. The rotating drum 30 having a substantially cylindrical shape and rotates in one direction about a central axis C30 which is along the CD direction. The ultrasonic processing units 60, 60... rotates about the central axis C30 together with the rotating drum 30. Concerning the substrate 1a which is being transported from an upstream process, the pair of guide rolls 90a and 90b transports the substrate 1a to the downstream process while winding the substrate 1a on the outer circumferential face 30s of the rotating drum 30 through a predetermined range Rw in the rotation direction Dc30 (FIG. 8).

The rotating drum 30 is driven and rotated at substantially the same circumferential speed value (m/min) as the transport speed value (m/min) of the substrate 1a which is being transported from the upstream process. Accordingly, the substrate 1a is being transported along the outer circumferential face 30s of the rotating drum 30 while the substrate 1a being wound on the outer circumferential face 30s, substantially without relative slippage to the substrate 1a. Then, after the substrate 1a has moved out of the predetermined range Rw, the substrate 1a is separated away from the outer circumferential face 30s and is transported to the downstream process.

In the description below, for the purpose of explanation, it is assumed that the circumferential speed value (m/min) of the rotating drum 30 is constant. That is, in an actual production line, the circumferential speed value (m/min) of the rotating drum 30 will fluctuate. For example, when starting or stopping the production line, or when being in a sudden trouble, the rotating drum 30 rotates at a circumferential speed value which is different from a constant circumferential speed value (m/min), which is a normal speed value. But, most of the available time for producing the diapers 1, the rotating drum 30 rotates at a constant circumferential speed value (m/min), which is the abovementioned normal speed value. On the other hand, the rotating drum 30 is rotating at an unsteady speed value, for a very short time like when starting the production line. Since the description based on the foregoing assumption will not disturb understanding the concept of the invention, the description is made below based on the foregoing assumption.

As shown in FIGS. 3 and 5, the ultrasonic processing units 60, 60... are provided at every predetermined angle (e.g. 90°) in the rotation direction Dc30 of the rotating drum 30. Each ultrasonic processing unit 60 includes a horn 61 and a roller-like anvil 71(corresponding to the roller member). The horn 61 vibrates ultrasonically and is arranged on the outer circumferential face 30s of the rotating drum 30 not to move relative to the outer circumferential face 30s. The anvil 71 is arranged outside with respect to the horn 61 in the rotation-radius direction Dr30 of the rotating drum 30 so that the substrate 1a is sandwiched between the anvil 71 and the horn 61.

Here, the horn 61 is in a rail-like shape extending in the CD direction, and roller-like anvil 71 (hereinafter referred to as an anvil roller 71) is capable of rolling on such a rail-like horn 61 (corresponding to the rail-like member). And, the anvil roller 71 is capable of reciprocating in the CD direction with respect to a part 1ap of the substrate 1a which is placed on the horn 61. Accordingly, during the reciprocation, ultrasonic energy is selectively applied from the horn 61 to a part 1ap of the substrate 1a which is sandwiched between the anvil roller 71 and the horn 61. Consequently, a welded part 14 is formed in the part 1ap of the substrate 1a.

The configuration of the ultrasonic welding apparatus 20 will be described in detail below.

As shown in FIG. 3, the main body of the rotating drum 30 is a cylinder, and its cross section in which its normal direction is the CD direction is in a circular shape, for example. At the circle center, which is the center of this cross section, a shaft member 31 is provided on the abovementioned central axis C30 in a concentric and integrated manner. In addition, the shaft member 31 is supported rotatably by the bearing member 31brg shown in FIG. 6 while the axial direction of the shaft member 31 being oriented in the CD direction. Accordingly, the rotating drum 30 is capable of rotating about the abovementioned central axis C30.

The rotating drum 30 is provided with rotational force by the servomotor 30M, which is a driving source, through a suitable rotational-force transmission mechanism. Accordingly, the rotating drum 30 is driven and rotated in one direction.

For example, in the example of FIG. 6, a so-called belt-type transmission device is used as the rotational-force transmission mechanism. That is, in the belt-type transmission device, an endless timing belt 30TB is wound around a pulley 31PL and a pulley 30MPL; the pulley 31PL is provided on the one end section 31eb of the shaft member 31 in a concentric and integrated manner, and the pulley 30MPL is provided on the driving rotational shaft of the servomotor 30M in a concentric and integrated manner. Thereby, driving rotational force generated by the servomotor 30M is transmitted to the shaft member 31, which serves as the central axis C30 of the rotating drum 30. Accordingly, the rotating drum 30 is driven and rotated by the servomotor 30M. However, rotational-force transmission mechanism is not limited thereto. For example, it is acceptable that the abovementioned pulleys 31PL and 30MPL are respectively replaced with gears to configure the rotational-force transmission mechanism with a group of gears. In this example, though the cross section of the rotating drum 30 is in a circular shape, the invention is not limited thereto. For example, the shape of the cross section may be a polygon, for example, a regular polygon having more corners than the number of the ultrasonic processing units 60.

As shown in FIG. 5, a plurality (e.g. four) of the ultrasonic processing units 60, 60... are provided at every predetermined angle in the rotation direction Dc30 of the rotating drum 30. The predetermined angle is set to an angle in which the length in the rotation direction Dc30 on the outer circumferential face 30s of the rotating drum 30 is substantially equal to a length corresponding to a single diaper. In the example of FIG. 5, the predetermined angle is set to 90°. Accordingly, the number of the ultrasonic processing units 60, 60... which are provided is four. The servomotor 30M as a driving source is controlled by a control section (not shown) such as a computer, a programmable logic controller (PLC) or the like, and the controlling is performed so that the rotating drum 30 rotates by the predetermined angle as the substrate 1a is fed from the upstream process by a length corresponding to a single diaper. Accordingly, each to-be-welded part 1e of the substrate 1a is correlated to one of the ultrasonic processing units 60 and the ultrasonic welding process is performed. Such a rotation is realized, for example, by controlling the position of the abovementioned servomotor 30M based on synchronization signals.

The synchronization signals are outputted from a rotation detection sensor (not shown; e.g. a rotary encoder), which measures the transport amount of the substrate 1a in, for example, an apparatus which serves as a reference in the production line (e.g. a rotary die cutter apparatus which forms the leg opening 1LH of FIG. 2A by stamping). Such a synchronization signal is a rotational angle signal expressed by, for example, defining a transport amount corresponding to a single product diaper (substantially equal to the foregoing product pitch P1) as a unit transport amount and allocating the rotational angle values from 0° to 360° in proportion to the transportation amount. When the transportation is performed by an amount of the diaper, each of the rotational angle values between 0° and 360° is outputted repeatedly and periodically. However, the synchronization signals are not limited to the rotational angle signals. For example, as the synchronization signals, digital signals may be used which is obtained by allotting each of digital values for 0-8191 to the above-mentioned unit transport amount in proportion to the transport amount.

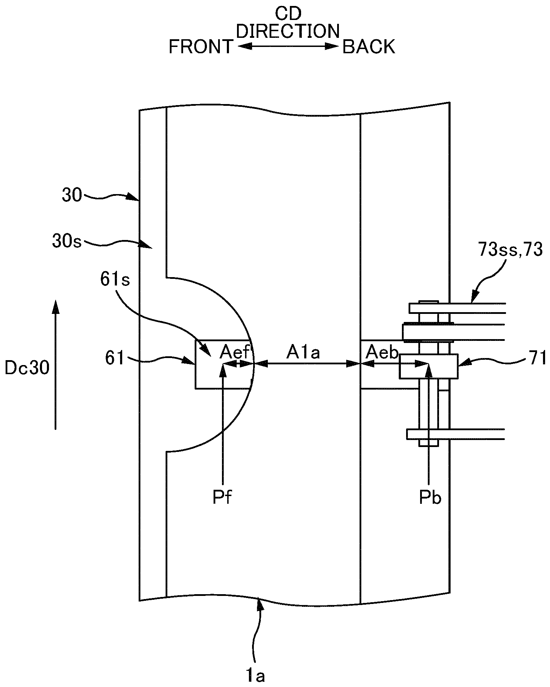

As illustrated in FIGS. 3 and 5, each ultrasonic processing unit 60 includes the rail-like horn 61 and the anvil roller 71. The horn 61 extends along the CD direction and is fixed to the column 41 (to be described later) so as not to move relative to the outer circumferential face 30s of the rotating drum 30. The anvil roller 71 is provided so as to reciprocate in the CD direction while rolling on the horn 61. As shown in FIGS. 3, 5 and 6, the horn 61 includes a substantially flat surface 61s facing outwards the rotation-radius direction Dr30 of the rotating drum 30, and on the substantially flat surface 61s, the anvil roller 71 rolls. The substantially flat surface 61s serves as the oscillating surface 61s that vibrates ultrasonically. The oscillating surface 61s is fixed to the outer circumferential face 30s of the rotating drum 30 in a state in which the surface 61s coincides with the outer circumferential face 30s, or in which the surface 61s slightly protrudes outwards in the rotation-radius direction Dr30. The length of the oscillating surface 61s in the CD direction is designed to be a dimension in which the oscillating surface 61s extends beyond both sides of the substrate 1a in the CD direction, the substrate 1a being wound around the outer circumferential face 30s of the rotating drum 30 (e.g. see FIG. 10). Accordingly, based on reciprocation of the anvil roller 71 in the CD direction, the welded part 14 can be formed through the entire length of the substrate 1a in the CD direction.

The horn 61 is connected to an oscillator via a booster and a converter (all of them are not shown) . The oscillator has an electric circuit, and the electric circuit generates electric signals having a certain frequency from 20kHz to 35kHz when power is supplied from a suitable power supply. The converter converts the electric signals having the certain frequency which has been sent from the oscillator, into mechanical vibration having the same frequency, by means such as a piezo element. The booster amplifies the mechanical vibration sent from the converter, and transmits to the horn 61. Accordingly, the oscillating surface 61s of the horn 61 vibrates ultrasonically in the direction normal to the surface 61s.

Here, ultrasonic energy is applied to the substrate 1a using ultrasonic vibration of the oscillating surface 61s of the horn 61. If the frequency of the ultrasonic vibration is constant, the amount (J) of the ultrasonic energy can be changed by any of the following methods: changing the amplitude of the ultrasonic vibration; or changing the magnitude (N) of force at which the substrate 1a is sandwiched between the oscillating surface 61s and the anvil roller 71 (hereinafter referred to as the sandwiching force). For example, if the magnitude (N) of sandwiching force is constant, the resistance to the vibration increases/decreases in conjunction with increase/decrease of the amplitude. Consequently, the power consumption of the oscillator increases/decreases. Since most of the power consumption that is applied to the substrate 1a as ultrasonic energy, ultrasonic energy that is applied to the substrate 1a increases/decreases in conjunction with increase/decrease of the amplitude. On the other hand, if the amplitude is constant, the resistance to the vibration increases/decreases in conjunction with increase/decrease of the magnitude (N) of sandwiching force. Consequently, the power consumption of the oscillator also increases/decreases, and most of the power consumption that is applied to the substrate 1a as ultrasonic energy. That is, ultrasonic energy that is applied to the substrate 1a increases/decreases in conjunction with increase/decrease of the magnitude (N) of sandwiching force.

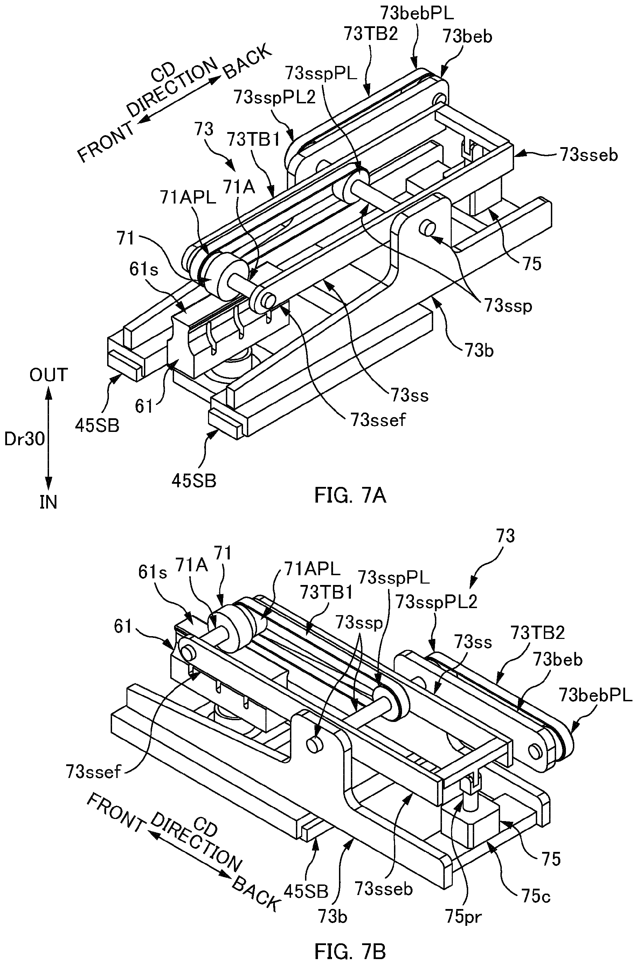

In the former case, changing the amplitude can be made by an oscillator. That is, the abovementioned oscillator is capable of changing the amplitude of the ultrasonic vibration to any value, based on control signals transmitted from the ultrasonic-energy regulating unit (not shown), the ultrasonic-energy regulating unit being composed of a computer, a PLC, or the like. And, in the latter case, changing the magnitude (N) of sandwiching force can be made by an air cylinder 75 (to be described later; see FIGS. 7A and 7B) provided in the anvil roller 71. This will be described later. In this example, the oscillator is capable of regulating the amplitude of the ultrasonic vibration (the distance from the balanced position to the maximum displacement) to any value between 0-30 µm. However, the regulatable range of the amplitude is not limited thereto.

On the other hand, as mentioned with reference to FIGS. 5 and 6, the anvil roller 71 faces the oscillating surface 61s of the horn 61, and is arranged outside with respect to the oscillating surface 61s in the rotation-radius direction Dr30 of the rotating drum 30. The anvil roller 71 is provided so as to reciprocate in the CD direction while rolling on the oscillating surface 61s. For example, the reciprocation of the anvil roller 71 is realized as follows.

As shown in FIGS. 3, 5 and 6, inside the rotating drum 30, a column 41 having a polygonal tubular shape is provided coaxially with the foregoing shaft member 31, which serves as the central axis C30 of the rotating drum 30. Most part of the column 41 is accommodated inside the rotating drum 30, and the one end section 41eb of the column 41 in the CD direction protrudes beyond the rotating drum 30. The column 41 is connected to and integrated with the shaft member 31 of the rotating drum 30 using a connecting member (not shown). Accordingly, the column 41 rotates about the central axis C30 together with the rotating drum 30. In the description below, concerning the CD direction, a direction toward which the column 41 protrudes beyond the rotating drum 30 is called as "back", and the opposite direction is called as "front".

As shown in FIG. 5, a cross section of the column 41 (a cross section in which its normal direction is the CD direction) is a regular polygon having the same total corners as the ultrasonic processing units 60, 60..., for example. Accordingly, the column 41 is a tubular body having the same number of wall sections 41w, 41w... as the ultrasonic processing units 60, 60.... In the example of FIG. 5, the column 41 includes four ultrasonic processing units 60, 60..., and therefore the column 41 has a square cross section. In other words, the column 41 is a square tubular body having four wall sections 41w, 41w.... The wall sections 41w respectively correlate to the ultrasonic processing units 60 one by one. That is, each wall section 41w includes a linear guide 45 for reciprocating the anvil roller 71 of the ultrasonic processing unit 60 in the CD direction. As shown in FIG. 6, the linear guide 45 includes: rail 45R which is fixed to the wall section 41w and extends in the CD direction; and sliding blocks 45SB and 45SB which are engaged with the rail 45R so as to be able to slide towards both sides in the CD direction. A support unit 73, which supports the anvil roller 71, is fixed to the sliding blocks 45SB and 45SB.

FIGS. 7A and 7B are diagrams illustrating the support unit 73. FIG. 7A is a schematic perspective view of the support unit 73 as viewed obliquely from front above and outside in the rotation-radius direction Dr30, and FIG. 7B is a schematic perspective view of the support unit 73 as viewed obliquely from back and outside in the rotation-radius direction Dr30.

The support unit 73 includes: a base section 73b fixed to the sliding blocks 45SB, 45SB...; and a seesaw-like member 73ss which is supported by the base section 73b so as to be capable of oscillating and extends in the CD direction. Here, the seesaw-like member 73ss is supported by the base section 73b with a support shaft 73ssp so as to be capable of oscillating, the support shaft 73ssp being provided at substantially the center in the CD direction. That is, the front-end part 73ssef and the back-end part 73sseb of the seesaw-like member 73ss are each capable of oscillating in the rotation-radius direction Dr30 of the rotating drum 30. More specifically, the front-end part 73ssef and the back-end part 73sseb move in nearly opposite directions to each other. In the front-end part 73ssef, the abovementioned anvil roller 71 is supported rotatably. On the other hand, a double-acting air cylinder 75 is provided in the back-end part 73sseb as a driving source for causing the seesaw-like member 73ss to oscillate, for example.

As shown in FIG. 7B, the air cylinder 75 includes: a cylinder section 75c; a piston (not shown) which is capable of sliding inside the cylinder section 75c and which partitions the cylinder section 75c to form two pressure chambers; and a piston rod 75pr which is capable of coming out of and in the cylinder section 75c and which is integrated with the piston. The tip end of the piston rod 75pr is connected to the back-end part 73sseb of the seesaw-like member 73ss, and the cylinder section 75c is fixed to the base section 73b. Accordingly, by controlling the pressure (MPa) of compressed air being supplied to each of two pressure chambers, it is possible, through movement of the piston rod 75pr, to press the anvil roller 71 against the oscillating surface 61s of the horn 61 and to separate the anvil roller 71 from the oscillating surface 61s.

For example, if one pressure chamber is opened to the atmosphere and compressed air is supplied to the other pressure chamber, the substrate 1a can be sandwiched between the anvil roller 71 and the oscillating surface 61s of the horn 61. In addition, if the supply pressure (MPa) of the compressed air changes, it is possible to regulate the magnitude (N) of the sandwiching force for the substrate 1a. It should be noted that a mechanism for regulating the supply pressure (MPa) of compressed air which is supplied to the air cylinder 75 is exemplified by a configuration including pressure-regulator valves (not shown) and directional control valves (e.g. solenoid valves; not shown), the pressure-regulator valves being respectively provided in channels through which compressed air is supplied to the pressure chambers, the directional control valves respectively switching connection and disconnection of the supply channels to a compressed-air source (not shown). However, the invention is not limited thereto.

On the other hand, rotation of the column 41 generates driving force for reciprocating the support unit 73 of the anvil roller 71 in the CD direction. That is, the column 41 rotates in the rotation direction Dc30 in an integrated manner with the rotating drum 30, and the ultrasonic welding apparatus 20 has a cam mechanism which drives the support units 73 by converting the rotation into reciprocation in the CD direction and by transmitting to the support units 73.

As shown in FIG. 6, such a cam mechanism has, for example, a cylindrical member 51 that is inserted into the column 41 in a coaxial manner with the column 41, and the cylindrical member 51 is fixed to a suitable support member 55 on the ground GND side so as to be unable to rotate. A ribbed cam 51r is provided on the outer circumferential face 51s of the cylindrical member 51, and a pair of cam followers 53 and 53 are provided in the base section 73b of the support unit 73. The abovementioned ribbed cam 51r is sandwiched between the pair of cam followers 53 and 53 in the front-back direction and are engaged with the cam followers 53 and 53. The ribbed cam 51r is provided in an endless manner continuously in the rotation direction Dc30 of the rotating drum 30. Further, the position of the cam 51r in the CD direction is changing according to the position of the cam 51r in the rotation direction Dc30, and a cam curve is therefore set. The setting of the cam curve determines the reciprocation of the support unit 73.

For example, in this example, under the condition that the circumferential speed value (m/min) of the rotating drum 30 is constant, the abovementioned cam curve is set so that, in both of the forward path and the return path of reciprocation of the support unit 73, the support unit 73 moves at the same constant travel speed value (m/min) as each other.

More specifically, as shown in schematic front view of FIG. 8, within a winding angular range Rw in which the substrate 1a is wound around the rotating drum 30, a first angular range Rw1 and a second angular range Rw2 are defined, and these angular ranges have the same magnitude and do not overlap each other. Here, forward movement, movement in the forward path, is allocated to a part of the cam curve which corresponds to the first angular range Rw1. For this purpose, that part has a shape in which the position of the ribbed cam 51r in the CD direction shifts forward in proportion to the change of the position of the ribbed cam 51r in the rotation direction Dc30. Accordingly, when the support unit 73 passes the first angular range Rw1, the support unit 73 and the accompanying anvil roller 71 move at a constant travel speed value (m/min) from a backward limit Pb to a forward limit Pf, the backward limit Pb being provided at the back in the CD direction (FIG. 6), the forward limit Pf being provided at the front (FIG. 6). Furhter, backward movement, movement in the return path, is allocated to a part of the cam curve which corresponds to the second angular range Rw2. For this purpose, that part has a shape in which the position of the ribbed cam 51r in the CD direction shifts backward in proportion to the change of the position of the ribbed cam 51r in the rotation direction Dc30. Accordingly, when the support unit 73 passes the second angular range Rw2, the support unit 73 and the accompanying anvil roller 71 move from the forward limit Pf to the backward limit Pb at the same travel speed value (m/min) as in the forward path.

In the example of FIG. 8, the first angular range Rw1 and the second angular range Rw2 are provided being adjacent to each other. Accordingly, the anvil roller 71 which has reached the forward limit Pf as a result of the forward movement promptly turns and performs the backward movement. However, the invention is not limited thereto. For example, the anvil roller 71 may performs the backward movement after the anvil roller 71 stops for some period of time at the forward limit Pf.

In a state in which the anvil roller 71 is positioned at the backward limit Pb, the anvil roller 71 has completely finished crossing the substrate 1a, and is not in contact with the substrate 1a (see FIG. 10, for example) . The abovementioned cam curve is set so that the anvil roller 71 stays at the backward limit Pb within an angular range Rw3 in the rotation direction Dc30, the angular range Rw3 being a range except for the first and second angular ranges Rw1 and Rw2. Accordingly, if a position of the initial end of the winding angular range Rw of the substrate 1a is defined as a winding start position Pws and a position of the final end of the winding angular range Rw is defined as a winding end position Pwe, the anvil roller 71 is positioned, at the backward limit Pb at both of the winding start position Pws and the winding end position Pwe. This allows the anvil roller 71 not to interrupt the following operations: winding operation of the substrate 1a to the outer circumferential face 30s of the rotating drum 30; and the releasing operation of the substrate 1a from the outer circumferential face 30s.

The anvil roller 71 rotates according to its reciprocation in the CD direction. That is, during the forward movement, the roller 71 is rotating forward at substantially the circumferential speed value (m/min) as the travel speed value (m/min) so as to roll forward. And, during the backward movement, the roller 71 is rotating backward at substantially the circumferential speed value (m/min) as the travel speed value (m/min) so as to roll backward. Accordingly, the anvil roller 71 is rolling on the substrate 1a and moving in the CD direction, substantially without relative slippage to the substrate 1a.

The foregoing rotation movements of the anvil roller 71 may be made without a driving source. For example, as shown in FIGS. 7A and 7B, whereas the anvil roller 71 is supported by the support units 73 through suitable bearing member (not shown), the anvil roller 71 is capable of rotating at an extremely small rotational resistance. And, the foregoing air cylinder 75 presses the anvil roller 71 against the oscillating surface 61s of the horn 61 via the substrate 1a. Accordingly, according to the reciprocation of the anvil roller 71, the anvil roller 71 obtains rotational force from the substrate 1a and rotates. Then, the anvil roller 71 is rotating as a follower.

However, the foregoing rotation of the anvil roller 71 as a follower leads a problem of reliability of the rotation. In the example of FIGS. 7A and 7B, for a purpose of ensuring the rotation of the anvil roller 71, there is provided a movement converting mechanism which converts the reciprocation of the support unit 73 into rotational movement and transmits it the anvil roller 71. The movement converting mechanism is a so-called belt-type transmission mechanism.

That is, a pulley 71APL is fixed to a shaft section 71A which is integrated and concentric with the anvil roller 71, and another pulley 73sspPL is rotatably supported by the support shaft 73ssp of the seesaw-like member 73ss. An endless timing belt 73TB1 is wound around these pulleys 71APL and 73sspPL. To the latter pulley 73sspPL, a pulley 73sspPL2 is integrated and fixed in a concentric manner. In addition, another pulley 73bebPL is rotatably supported in a back-end part 73beb of the base section 73b of the support unit 73. Another endless timing belt 73TB2 is wound around these pulleys 73sspPL2 and 73bebPL. In addition, a part of the timing belt 73TB2 is connected to the column 41 through a connecting member.

Accordingly, when the support unit 73 is moving in the CD direction relative to the column 41, the anvil roller 71 is rotating by the amount of this movement. That is, when the support unit 73 moves forward, the anvil roller 71 rotates in the forward direction by the same amount as the amount of this forward movement. On the other hand, when the support unit 73 moves backward, the anvil roller 71 rotates in the backward direction by the same amount as the amount of this backward movement. This allows the anvil roller 71 to reliably roll on the substrate 1a during its reciprocation in the CD direction, substantially without relative slippage to the substrate 1a. It should be noted that this relates to forming the welded parts 14 in a pattern the anvil roller 71 (to be described later).

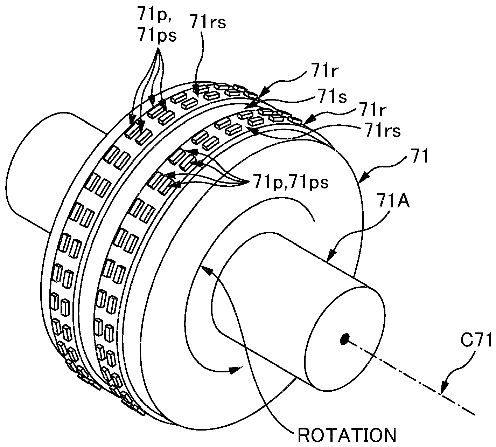

FIG. 9 is a schematic perspective view of the anvil roller 71. On the circumferential face 71s of the anvil roller 71, a plurality of protrusions 71p, 71p... are provided for forming the welded parts 14 in a pattern on the substrate 1a. Specifically speaking, on the circumferential face 71s of the anvil roller 71, two endless ribs 71r and 71r are provided in a direction along the rotational axis C71 of the anvil roller 71, and the ribs 71r and 71r extend through the entire length in its circumferential direction. On the top surface 71rs of each rib 71r, a plurality of island-shaped protrusions 71p, 71p... are placed in a pattern in which there is a certain regularity. And, the top surfaces 71rs of the ribs 71r form low-compressed parts in the substrate 1a, and the top surfaces 71ps, 71ps... of the plurality of protrusions 71p, 71p... form high-compressed parts at a compression ratio larger than that for the low-compressed parts. Accordingly, the welded parts 14 are formed in a pattern in which there are a plurality of high-compressed parts.

As mentioned above, the anvil roller 71 is rotating according to reciprocation in the CD direction, and is rolling, substantially without relative slippage to the substrate 1a. Accordingly, the protrusions 71p, 71p... of the anvil roller 71 are designed to abut the substrate 1a at the same positions in both of the forward path and the return path of the reciprocation. In other words, it is assumed that the high-compressed part formed in the return path completely superpose the high-compressed parts formed in the forward path. In this assumption, the high-compressed parts formed in the return path coincide with the high-compressed parts formed in the forward path even if the amount (J/m) of ultrasonic energy applied per unit length in the CD direction is equal between in the forward path and in the return path. Consequently, the appearance of the welded parts 14, which includes the high-compressed parts, does not become bad.

But, in actual process, it is possible that the substrate 1a is slightly shifted between the forward path and the return path, and it is also possible that the rotation of the anvil roller 71 do not completely follow the reciprocation in the CD direction. In this case, the high-compressed parts, which should be formed in the forward path and the return path so as to coincide, may be formed being shifted. Accordingly, the appearance of the welded part 14 becomes bad, and this decreases the commodity value of the diaper 1.

In the ultrasonic welding apparatus 20, a section in the CD direction in which the anvil roller 71 crosses over the substrate 1a as shown in FIG. 10 is defined as a "crossing section A1a" (corresponding to the predetermined section). Concerning the reciprocation of the anvil roller 71 in the CD direction, ultrasonic energy is applied when the anvil roller 71 is crossing the crossing section A1a. The amount (J/m) of the applied ultrasonic energy per unit length in the CD direction is different between the forward path and the return path of the reciprocation.

For example, in the reference example, ultrasonic energy is applied across the entirety of the crossing section A1a in the forward path while maintaining the amount (J/m) of the ultrasonic energy per unit length in the CD direction to be constant. Also, in the return path, ultrasonic energy is applied across the entirety of the crossing section A1a while maintaining the amount (J/m) of the ultrasonic energy per unit length in the CD direction to be constant. But, the energy amount (J/m) in the forward path is larger than the energy amount (J/m) in the return path.

Accordingly, in the forward path, the substrate 1a can undergo considerably the ultrasonic welding process, and in the return path, ultrasonic welding process to the substrate 1a can be reduced. That is, in the return path, the welded part 14 is formed less firmly or is not formed at all. Therefore, even if the high-compressed parts of the welded part 14 formed in the forward path do not coincide the high-compressed parts of the welded part 14 formed in the return path, the positional variation can be inconspicuous.

The changing the amount (J/m) of applied ultrasonic energy between the forward path and the return path is made by controlling of the ultrasonic-energy regulating unit to the oscillator. The detailed description is as follow.

First, the ultrasonic-energy regulating unit is always monitoring the position of the support unit 73 in the CD direction during its reciprocation. The monitoring is performed, for example, indirectly based on signals outputted from a rotary encoder (not shown), which serves as a rotation detection sensor that detects the rotational angle of the rotating drum 30. That is, as mentioned above, the relation between the positions of the rotating drum 30 in the rotation direction Dc30 and in the CD direction is uniquely predetermined based on the cam curve of the ribbed cam 51r. Accordingly, if the position of the support unit 73 in the rotation direction Dc30 is determined, it is possible to recognize the current position of the support unit 73 in the CD direction. On the other hand, the position of the support unit 73 in the rotation direction Dc30 can be detected the based on the rotational angle value (0° to 360°) indicated by a signal outputted from the encoder.

For this purpose, the following data are stored in advance in the memory of the regulating section: the values of the rotational angles corresponding to the positions at which the anvil roller 71 is located in the forward path immediately before the anvil roller 71 finishes crossing the abovementioned crossing section A1; the values of the rotational angles corresponding to the positions at which the anvil roller 71 is located in the forward path when the anvil roller 71 has finished crossing the crossing section A1a; the values of the rotational angles corresponding to the positions at which the anvil roller 71 is located in the forward path immediately after the anvil roller 71 has finished crossing the crossing section A1a; or the like. The regulating section detects that the rotational angle value indicated by a signal outputted from the encoder becomes equal to or larger than a rotational angle value in the memory. Then, simultaneously or when a predetermined time has elapsed after the detection, the regulating section changes the amplitude to a smaller amplitude for the return path.

Similarly, the following data are stored in advance in the memory of the regulating section: the values of the rotational angles corresponding to the positions at which the anvil roller 71 is located in the return path immediately before the anvil roller 71 finishes crossing the abovementioned the crossing section A1a; the values of the rotational angles corresponding to the positions at which the anvil roller 71 is located in the return path when the anvil roller 71 has finished crossing the crossing section A1a; the values of the rotational angles corresponding to the positions at which the anvil roller 71 is located in the return path immediately after the anvil roller 71 has finished crossing the crossing section A1a; or the like. The regulating section detects that the rotational angle value indicated by a signal outputted from the encoder becomes equal to or larger than a rotational angle value in the memory. Then, simultaneously or when a predetermined time has elapsed after the detection, the regulating section changes the amplitude to a larger amplitude for the forward path. This reliably allows an amplitude in the forward path to be larger than an amplitude in the return path.

The foregoing amplitude in the forward path is set, for example, to any value within a range from 15 µm to 30 µm. In this example, the amplitude is set to 30 µm. On the other hand, the amplitude in the return path is set, for example, to any value within a range from 0% to 50% of the amplitude in the forward path. In this example, the amplitude is set to 30% of the amplitude in the forward path. The foregoing setting makes it possible to reliably form the welded part 14 during the forward path and also makes it possible to reliably prevent formation of the welded part 14 during the return path. This enables to be further inconspicuous the positional variation between the welded part 14 made in the forward path and the welded part 14 made in the return path. It should be noted that the optimal value of the amplitude can be obtained as a result of actual experiments, etc.

In this example, the magnitude (N) of sandwiching force at which the substrate 1a is sandwiched between the oscillating surface 61s of the horn 61 and the anvil roller 71 is kept to be a constant value throughout the crossing section A1a, in both of the forward path and the return path. Accordingly, changing the amplitude realizes reliably that the amount (J/m) of ultrasonic energy applied during the return path is smaller than the amount (J/m) of ultrasonic energy applied during the forward path. The optimal value of the magnitude (N) of sandwiching force can be obtained as a result of actual experiments, etc.

In this example, the foregoing air cylinder 75 can separate the anvil roller 71 from the substrate 1a. But, as mentioned above, in the crossing section A1a, the horn 61 and the anvil roller 71 cooperate each other in both of the forward path and the return path so that the substrate 1a is sandwiched therebetween. That is, during both of the forward path and the return path, the horn 61 and the anvil roller 71 are in contact with the substrate 1a. In addition, the anvil roller 71, which can be separated from the substrate 1a, is not separated from the substrate 1a. Accordingly, the positional relation in the crossing section A1a between the anvil roller 71 and the horn 61 can be kept constant. This makes it possible to stabilize the ultrasonic welding process. The separating operation is required to be performed in small amounts of time, a part of a period of hundreds milliseconds, as mentioned above. It is therefore difficult to perform it, and the ultrasonic welding apparatus 20 becomes difficult to be achieved. But, in this example, since the anvil roller 71 is not separated from the substrate 1a, the ultrasonic welding apparatus 20 can be realized without any problem.

In the above description, the predetermined section in the CD direction on the outer circumferential face 30s of the rotating drum 30 is exemplified by "the crossing section A1a". As seen in FIG. 10, "the crossing section A1a" means "a section A1a corresponding to an area between the following positions of the substrate 1a, which does not meander and is wound around the outer circumferential face 30s of the rotating drum 30 at a design position: a position at which the substrate 1a starts crossing in the CD direction; and a position at which the substrate 1a ends the crossing". In the above description, the amplitude in the crossing section A1a is different between the forward path and the return path. However, the invention is not limited thereto. For example, the amplitude in a specific section (a part of the crossing section A1a; corresponding to the predetermined section) do not have to be different between the forward path and the return path. In other words, in the remaining section except for the specific section, the amplitude is not necessary to be different between the forward path and the return path. This enables to be inconspicuous the positional variation between the welded part 14 formed in the forward path and the welded part 14 formed in the return path, the welded parts 14 being formed in the part of the substrate 1a which corresponds to the abovementioned specific section.

Furthermore, a plurality of specific sections may be set so as not to overlap the crossing section A1a. For example, the position of the first specific section in the CD direction may be different from the position of second specific section in the CD direction. In this case, the amplitude of the ultrasonic vibration when the anvil roller 71 passes the first specific section is different between the forward path and the return path. In addition, the amplitude of the ultrasonic vibration when the anvil roller 71 passes the second specific section is different between the forward path and the return path. However, the amplitude in the first specific section is not necessary to be the same as the amplitude in the second specific section, and these amplitudes may be different.

### <<< First Modified reference example >>>

In the reference example mentioned above, by changing the amplitude of the ultrasonic vibration, the amount (J/m) of ultrasonic energy applied to the substrate 1a is different between the forward path and the return path. In this regard, in the first modified reference example, the amplitude is kept to be a constant value without changing between the forward path and the return path. And, instead thereof, a force at which the substrate 1a is sandwiched between the oscillating surface 61s of the horn 61 and the anvil roller 71, that is, the magnitude (N) of sandwiching force is changed between the forward path and the return path. Consequently, the amount (J/m) of ultrasonic energy is different between the forward path and the return path.

The rest of the configuration is substantially the same as that of the reference example. Therefore, mainly the difference will be described below. The same components as those of the reference example will be denoted by the same reference numerals, and the description thereof has been omitted.

First, in the first modified reference example, the ultrasonic-energy regulating unit controls the oscillator, and thereby the amplitude of the ultrasonic vibration of the oscillating surface 61s of the horn 61 is kept, for example, to a constant value of 30 µm. The regulating section controls the foregoing pressure-regulator valve for the air cylinder 75, which is provided in the support unit 73. Accordingly, in the forward path, the substrate 1a is sandwiched at a large sandwiching force (N) throughout the crossing section A1a. On the other hand, in the return path, the substrate 1a is sandwiched throughout the crossing section A1a at a smaller sandwiching force (N) than the sandwiching force in the forward path. As a result, the amount (J/m) of applied ultrasonic energy per unit length in the CD direction in the crossing section A1a of the forward path is larger than that in the crossing section A1a of the return path.

For example, the magnitude (N) of sandwiching force in the return path is set to any value within a range of 5% to 50% of the magnitude (N) of sandwiching force in the forward path. It is desirable that the magnitude in the return path is set to any value within a range of 5% to 30%. This enables the positional variation of the welded parts 14 to be further inconspicuous.

### <<< Second Modified reference example >>>

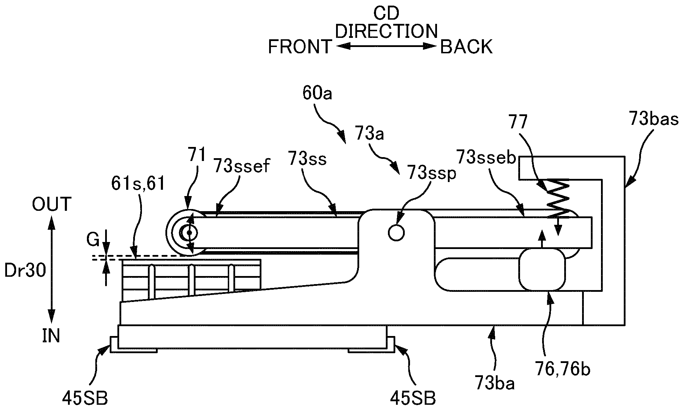

FIG. 11 is a schematic diagram of an ultrasonic processing unit 60a according to the second modified reference example as viewed in the rotation direction Dc30 of the rotating drum 30. In the second modified reference example, the size of the space G between the anvil roller 71 and the oscillating surface 61s of the horn 61 changes, and thereby, the amount (J/m) of ultrasonic energy is different between the forward path and the return path.

That is, in the second modified reference example, the amplitude is also a constant value and does not change between the forward path and the return path. But, the abovementioned size of the space G changes between the forward path and the return path. More specifically, in the forward path, the size of the space G is set to be small throughout the crossing section A1a. On the other hand, in the return path, the size of the space G is set throughout the crossing section A1a to be larger than the size of the space G in the forward path. Accordingly, the amount (J/m) of applied ultrasonic energy per unit length in the CD direction in the crossing section A1a of the forward path is larger than that in the crossing section A1a of the return path.

Changing the space G is realized, for example, by the following configuration. As shown in FIG. 11, in the second modified reference example, there are support units 73a each having substantially the same configuration as the support units 73 of the reference example. That is, the anvil roller 71 is supported rotatably by the front-end part 73ssef of the seesaw-like member 73ss. And, as mentioned above, the seesaw-like member 73ss is also rotatably supported by the support shaft 73ssp on the base section 73ba of the support unit 73a, the support shaft 73ssp being located at substantially the center in the CD direction. Accordingly, in the seesaw-like member 73ss, the front-end part 73ssef and the back-end part 73sseb can move in nearly opposite directions to each other. In other words, the seesaw-like member 73ss is capable of oscillating in a seesaw-like manner.

On the other hand, as driving sources for causing the seesaw-like member 73ss to oscillate, the support unit 73a of the second modified reference example includes: an air spring 76 that gives to the seesaw-like member 73ss a force in a direction in which the space G is reduced; and a leaf spring 77 that serves as elastic member and that gives to the seesaw-like member 73ss a force in a direction in which the space G is enlarged.

More specifically, the air spring 76 has a bag body 76b that is substantially sealed. The bag body 76b inflates when internally pressurized by an air supply, and deflates when internally depressurized by ejection of air. The bag body 76b is placed between the base section 73ba of the support unit 73a and the back-end part 73sseb of the seesaw-like member 73ss. And, the bag body 76b is in contact with the back-end part 73sseb from inside in the rotation-radius direction Dr30 of the rotating drum 30. The leaf spring 77 is supported by a suitable stay member 73bas, which is disposed of the base section 73ba of the support unit 73a so as to be in contact with the back-end part 73sseb of the seesaw-like member 73ss from outside in the rotation-radius direction Dr30. Accordingly, the leaf spring 77 gives to the back-end part 73sseb of the seesaw-like member 73ss an elastic force being directed inwards in the rotation-radius direction Dr30.

Accordingly, the bag body 76b is internally pressurized by such means as supplying air to the inside of the bag body 76b and increasing its supply pressure (MPa). Thereby, by inflation of the bag body 76b, the bag body 76b gives to the abovementioned the back-end part 73sseb a force being directed outwards the rotation-radius direction Dr30 of the rotating drum 30. When the magnitude of the force becomes larger than a force required to press and flatten the leaf spring 77 outwards in the rotation-radius direction Dr30, the seesaw-like member 73ss resists elastic force of the leaf spring 77 and simultaneously rotates so that the back-end part 73sseb moves outwards in the rotation-radius direction Dr30. And, the anvil roller 71 in the front-end part 73ssef moves inwards in the rotation-radius direction Dr30. Consequently, the space G between the oscillating surface 61s of the horn 61 and the anvil roller 71 is reduced.

On the other hand, the bag body 76b is internally depressurized by such means as decreasing supply pressure (MPa) at which air is supplied inside the bag body 76b. Thereby, by deflation of the bag body 76b, a force that the bag body 76b gives to the back-end part 73sseb is reduced. When the force becomes smaller than a force required to press and flatten the leaf spring 77 outwards in the rotation-radius direction Dr30, the seesaw-like member 73ss rotates so that the back-end part 73sseb moves inwards in the rotation-radius direction Dr30. And, the anvil roller 71 in the front-end part 73ssef moves outwards in the rotation-radius direction Dr30, and the space G between the horn 61 and the anvil roller 71 is consequently enlarged.

A mechanism which supplies compressed air to the bag body 76b of the air spring 76 and which also ejects air from the same is exemplified by a configuration in which a compressed-air source (e.g. a compressor; not shown) is connected to the bag body 76b through a supply channel (e.g. pipework; not shown), and in which a pressure-regulator valve (not shown) is arranged in the supply channel. In this configuration, the ultrasonic-energy regulating unit controls the pressure-regulator valve, and thereby the size of the space G is changed. However, the mechanism for supplying and ejecting compressed air is not limited thereto.

In the description above, the elastic member that gives a force in a direction in which the space G is enlarged is exemplified by the leaf spring 77. However, the invention is not limited thereto. For example, a disc spring or a coiled spring may also be employed. In addition, a bar-like member which undergoes elastic deflection deformation may be employed. For example, the one end section of the bar-like member is in contact with the back-end part 73sseb of the seesaw-like member 73ss from outside in the rotation-radius direction Dr30, and the bar-like member is supported on two parts except for the one end section by the base section 73ba of the support unit 73a so as not to rotate. When a force outwards in the rotation-radius direction Dr30 is applied to the back-end part 73sseb from the air spring 76, the bar-like member undergoes elastic deflection deformation, and thereby the size of the space G is changed.

### <<< Third Modified reference example >>>

In the third modified reference example, because of changing the travel speed value (m/min) of the anvil roller 71 in the CD direction, the amount (J/m) of ultrasonic energy is different between the forward path and the return path. The detailed description is as follow.

First, the amplitude and the magnitude (N) of sandwiching force are kept to be the same value without being different between the forward path and the return path. But, the travel speed value (m/min) of the anvil roller 71 in the CD direction is different between the forward path and the return path. More specifically, in the forward path, the travel speed value (m/min) is set to be small throughout the crossing section A1a, and the anvil roller 71 therefore moves at a low speed in the CD direction. On the other hand, in the return path, the travel speed value (m/min) is set to be larger than the travel speed value (m/min) in the forward path throughout the crossing section A1a, and the anvil roller 71 therefore moves at a high speed in the CD direction. As a result, the amount (J/m) of ultrasonic energy applied in the forward path per unit length in the CD direction is larger than the amount (J/m) of ultrasonic energy applied in the return path.

The changing of the travel speed value (m/min) of the anvil roller 71 in the CD direction is realized by the setting of the cam curve. That is, in this example, instead of the ultrasonic-energy regulating unit, which is used in the reference example and is composed of a computer or the like, the foregoing ribbed cam 51r and cam followers 53 and 53 serve as the ultrasonic-energy regulating unit. The detailed description is as follow.

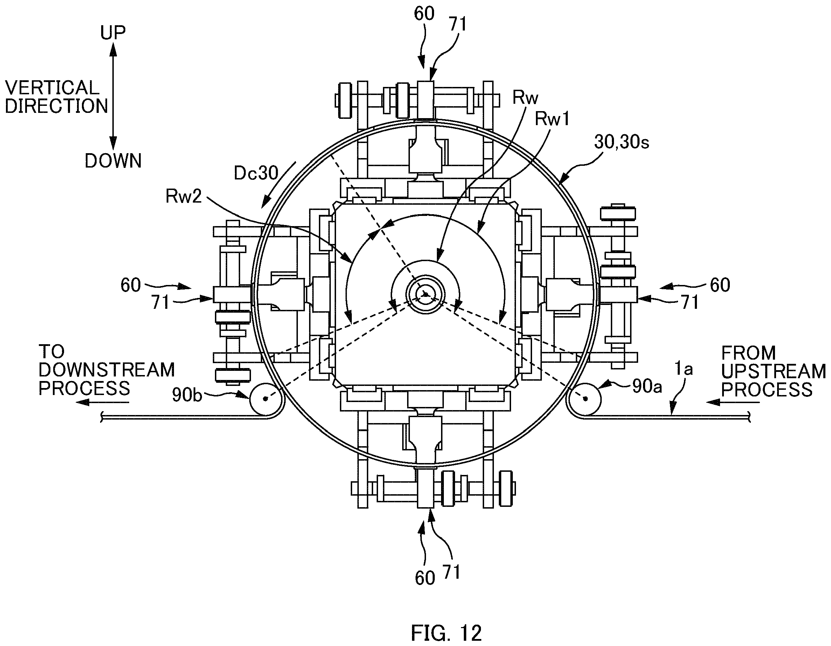

First, as mentioned above with reference to FIG. 8, in the cam curve of the cam 51r, the movement in the forward path (forward movement) is defined and corresponds to the first angular range Rw1 in the rotation direction Dc30 of the rotating drum 30. Also, in the cam curve, the movement in the return path (backward movement) is defined and corresponds to the second angular range Rw2 in the rotation direction Dc30. Accordingly, if the magnitude of the first angular range Rw1 is set to be larger than the magnitude of the second angular range Rw2 as shown in FIG. 12, the movement in the forward path is slower than the movement in the return path. That is, the travel speed value (m/min) in the forward path is set to be smaller than the travel speed value (m/min) in the return path.

In the reference example and the first to the third modified reference example, the rail-like horn 61 is arranged so as not to move relative to the rotating drum 30, and the roller-like anvil 71, which serves as the anvil roller 71, is arranged outside with respect to the horn 61 in the rotation-radius direction Dr30. However, the reference examples are not limited thereto. For example, the following configuration may be employed. That is, a rail-like anvil extending in the CD direction is fixed so as not to move relative to the rotating drum 30, and simultaneously a roller-like horn (hereinafter referred to as a horn roller) may be arranged outside with respect to the anvil in the rotation-radius direction Dr30. The configuration of the horn roller is the same as disclosed in Japanese Unexamined Patent Application Publication (Translation of PCT Application) No. 10-513128, and the configuration is known. Accordingly, the detailed description will be omitted.

### === First embodiment ===

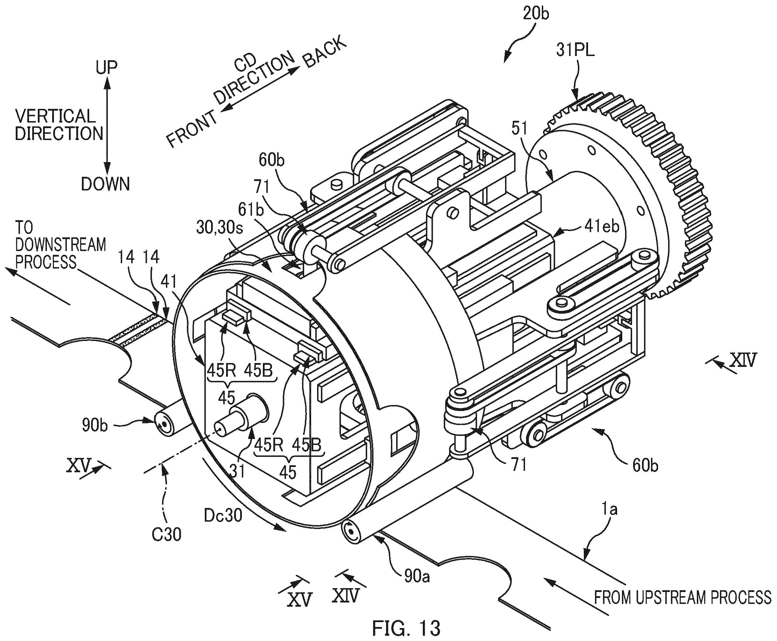

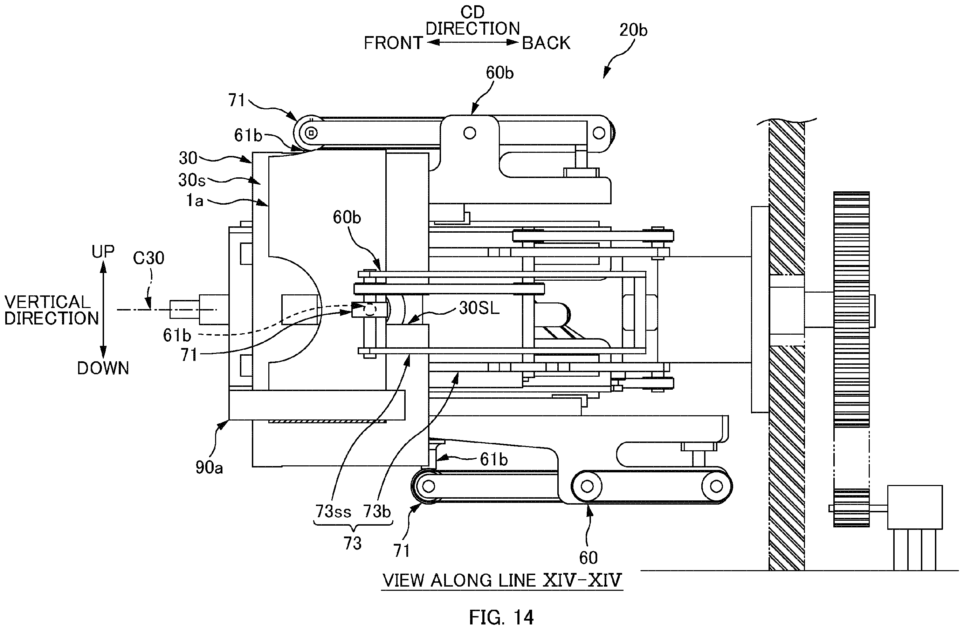

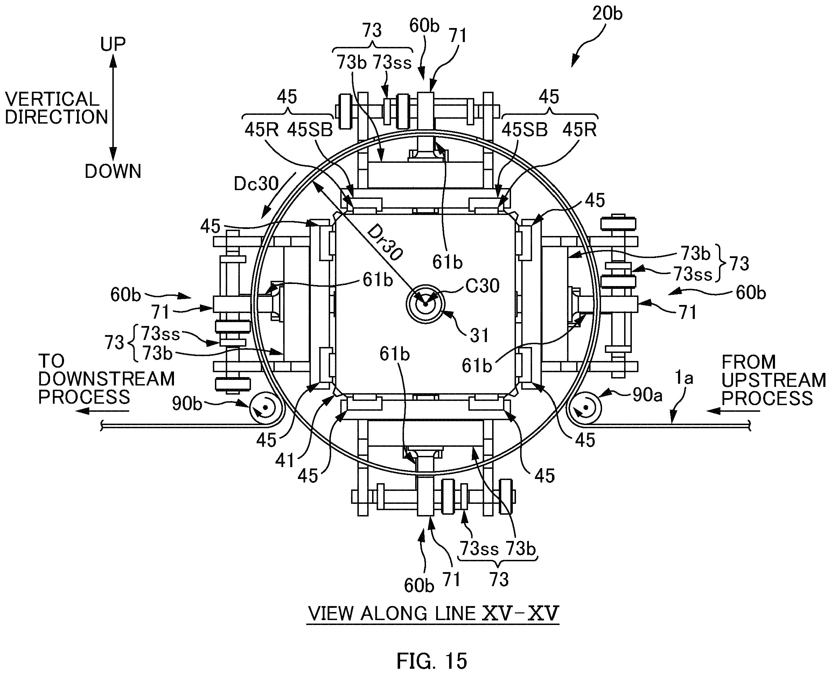

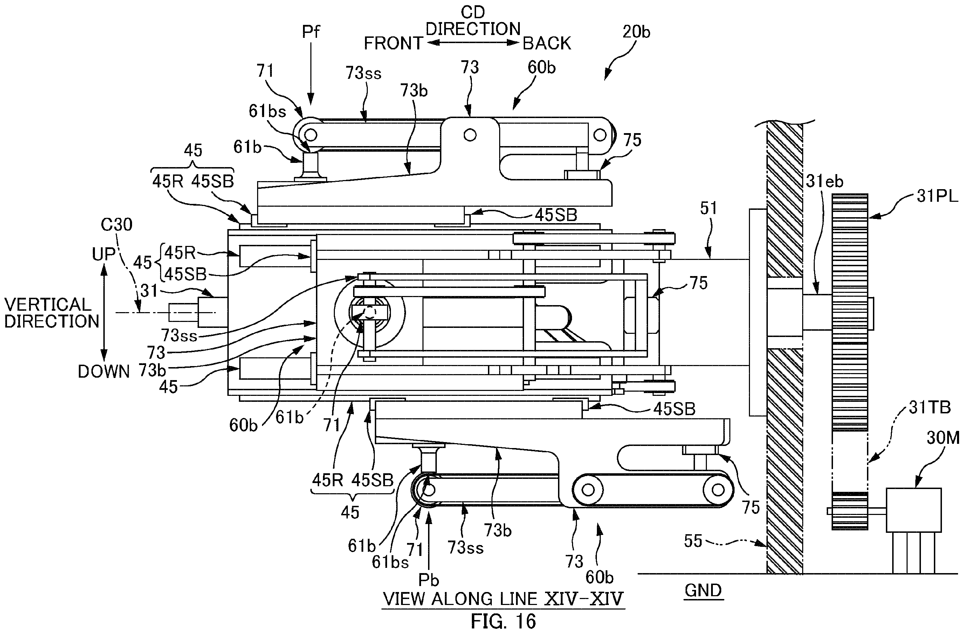

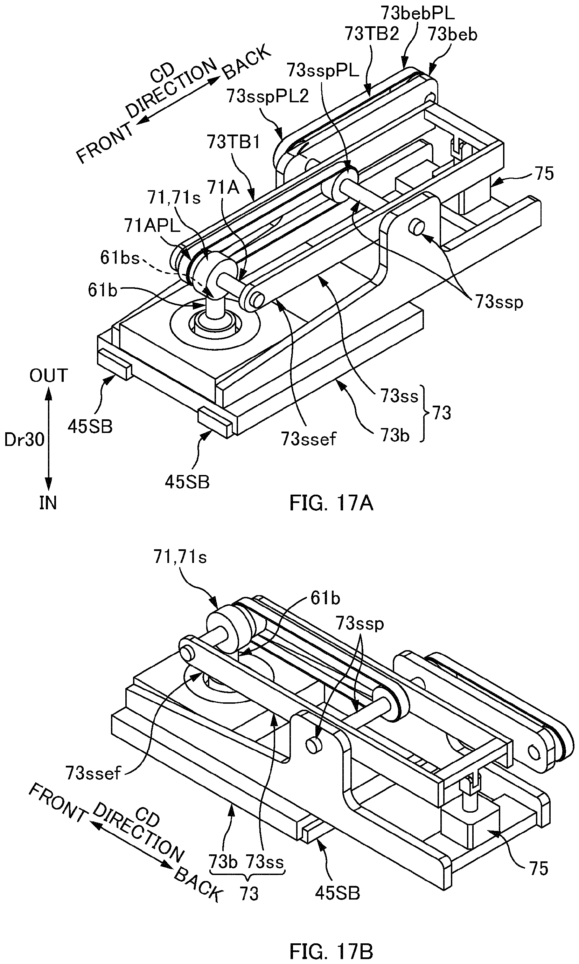

FIGS. 13 to 17B are diagrams illustrating the ultrasonic welding apparatus 20b according to the first embodiment. FIG. 13 is a schematic perspective view of the ultrasonic welding apparatus 20b as viewed obliquely from front above. FIG. 14 is a schematic side view along arrows XIV-XIV in FIG. 13, and FIG. 15 is a schematic front view along arrows XV-XV in FIG. 13. FIG. 16 is a schematic side view of the apparatus 20b in which the substrate 1a and the rotating drum 30 are removed from FIG. 14. FIG. 17A is a schematic perspective view of the ultrasonic processing unit 60b as viewed obliquely from front and outside in the rotation-radius direction Dr30, and FIG. 17B is a schematic perspective view of the unit 60b as viewed obliquely from back and outside in the rotation-radius direction Dr30.

In the reference example mentioned above, the horn 61 of the ultrasonic processing unit 60 is fixed to the column 41 so as not to move relative to the rotating drum 30, as shown in FIG. 5. And, the horn 61 do not reciprocate in the CD direction, as shown in FIG. 6. In this regard, the first embodiment is different from the reference example mainly because the horn 61b reciprocates in the CD direction in conjunction with the reciprocation of the anvil roller 71 in the CD direction, as shown in FIG. 16. The rest of the configuration is substantially the same as that of the reference example. Therefore, mainly the difference will be described below. The same components as those of the reference example will be denoted by the same reference numerals, and the description thereof has been omitted.

As shown in FIG. 16, in the first embodiment, in addition to the anvil roller 71, the horn 61b is also supported by the support unit 73. And, the horn 61b is also capable of reciprocating in the CD direction. The anvil roller 71 and the horn 61b are configured to cooperate each other so that the substrate 1a can be sandwiched therebetween. For example, in this example, the horn 61b is fixed to the base section 73b of the support unit 73. On the other hand, the anvil roller 71 is supported by the seesaw-like member 73ss. Accordingly, while ultrasonic energy being applied to the substrate 1a from the oscillating surface 61bs of the ultrasonically vibrating horn 61b, the anvil roller 71 and the oscillating surface 61bs of the horn 61b cooperate each other so that the substrate 1a is sandwiched therebetween. Simultaneously, both of the anvil roller 71 and the horn 61b reciprocate in the CD direction. Consequently, a welded part 14 along the CD direction is formed in the substrate 1a.

In the ultrasonic welding process to the substrate 1a, weld residue will adhere to and is accumulated on the horn 61b in particular; this is because the horn 61b does not rotate. But, in this example, in addition to the anvil roller 71, the horn 61b also reciprocates. Accordingly, during its reciprocation, the horn 61b can wipe successively the weld residue attached thereto, by being in contact with the substrate 1a and by sliding on the substrate 1a. This makes it possible to effectively prevent accumulation of weld residue on the horn 61b.

Sandwiching the substrate 1a between the anvil roller 71 and the oscillating surface 61bs of the horn 61b is realized by moving of the anvil roller 71 in the rotation-radius direction Dr30 of the rotating drum 30 through oscillation of the seesaw-like member 73ss, the oscillation being caused by mainly the air cylinder 75. Accordingly, it can be unnecessary to move the horn 61b the rotation-radius direction Dr30 of the rotating drum 30. This makes it possible to stabilize ultrasonic vibration of the horn 61b, which is composed of precision devices.

Also in the first embodiment, as shown in FIG. 14, the main body of the rotating drum 30 is a cylinder 30, which forms the outer circumferential face 30s. But, on the cylinder 30, slit-shaped notches 30SL are formed extending from back to front in the CD direction, and the notches 30SL correspond to reciprocation paths in the CD direction of each pair of the anvil roller 71 and the horn 61b. Accordingly, there is a space at the position of each notch 30SL. That is, the inside of the cylinder 30 communicates with the outside of the cylinder. Accordingly, the anvil roller 71 and the horn 61b can be reciprocating quickly in the CD direction without any interference with the cylinder 30, while the substrates 1a being sandwiched at the position of the notch 30SL between the anvil roller 71 and the horn 61b from both sides in the rotation-radius direction Dr30 of the rotating drum 30.

In the first embodiment, since the horn 61b reciprocates in the CD direction together with the anvil roller 71, it is not necessary for the horn 61b to have a rail-like shape extending in the CD direction as described in the reference example. That is, the shape of the horn 61b may be selected freely. Accordingly, in the first embodiment, the shape of the horn 61b is a substantially cylindrical body 61b whose axial direction is oriented in the rotation-radius direction Dr30 of the rotating drum 30, as shown in FIG. 17A. A circular side surface 61bs of the substantially cylindrical body 61b serves as the oscillating surface 61bs that vibrates ultrasonically, and the side surface 61bs faces outwards in the rotation-radius direction Dr30 of the rotating drum 30. Consequently, the horn 61b is fixed to the base section 73b of the support unit 73 with its side surface 61bs facing the circumferential face 71s of the anvil roller 71.

The basic configuration of the ultrasonic welding apparatus 20b according to the first embodiment is substantially the same as that of the apparatus disclosed in Japanese Unexamined Patent Application Publication No. 2013-193450. And, the detailed description thereof has been omitted.

Based on the foregoing description, it can be considered possible that persons skilled in the art realize the invention as long as it falls within the scope the appended claims, by applying the configurations of the first to third modified reference examples of the reference example to the ultrasonic welding apparatus 20b of the first embodiment. And, the description thereof has been omitted.

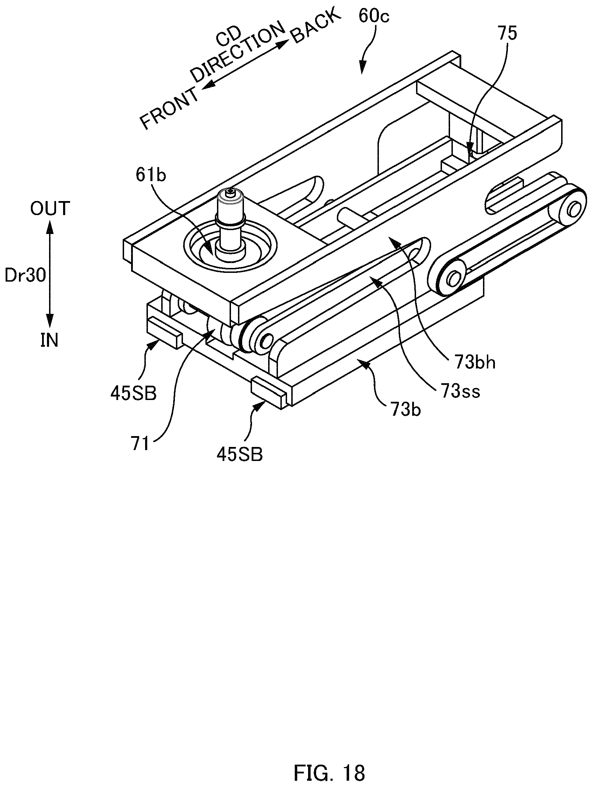

In addition, in the first embodiment, as shown in FIG. 17A, the horn 61b having a substantially cylindrical body is arranged inside with respect to the anvil roller 71 in the rotation-radius direction Dr30 of the rotating drum 30. However, the invention is not limited thereto. For example, the positional relation in the rotation-radius direction Dr30 may be inverted. That is, the anvil roller 71 may be arranged inside with respect to the horn 61b in the rotation-radius direction Dr30, the horn 61b being a substantially cylindrical body. In this case, the front-end part 73ssef of the seesaw-like member 73ss supports the horn 61b, and the base section 73b supports the anvil roller 71. However, in some cases, an ultrasonic processing unit may be the ultrasonic processing unit 60c shown in the schematic perspective view of FIG. 18. That is, in this example, the anvil roller 71 is supported by the seesaw-like member 73ss. The foregoing base section 73b includes a extending part 73bh, which extends outwardly in the rotation-radius direction Dr30 beyond the position of the seesaw-like member 73ss. And, the extending part 73bh supports the horn 61b. This configuration may be employed.

### === Other modified Reference Examples and Embodiments ===

While the embodiments of the invention are described above, the embodiments are for the purpose of elucidating the understanding of the invention and are not to be interpreted as limiting the invention. The scope of the invention is defined by the appended claims.

In the reference example, for the purpose of explanation, the description is made based on an assumption that the circumferential speed value (m/min) of the rotating drum 30 is constant. But, as mentioned above, the circumferential speed value of the rotating drum 30 may change in actual use. In this case, the amplitude of the ultrasonic vibration increases/decreases in conjunction with increase/decrease of the circumferential speed value (m/min) of the rotating drum 30. Concerning the amount (J/m) of ultrasonic energy applied per unit length in the CD direction, this makes it possible to maintain the relation between the forward path and the return path, without being affected by change of the circumferential speed value (m/min).

In the reference example, as shown in FIG. 9, the anvil roller 71 includes two ribs 71r and 71r on the circumferential face 71s, and a plurality of protrusions 71p, 71p... are provided in a pattern on the top surface 71rs of each rib 71r. However, the invention is not limited thereto. For example, the following configuration may be employed: two ribs along the CD direction are provided on the oscillating surface 61s of the rail-like horn 61 (see FIG. 6), and a plurality of protrusions are provided in a pattern on the top surface of each rib. In some cases, the abovementioned ribs 71r and protrusions 71p, 71p... may be provided on both of the anvil roller 71 and the horn 61. The rib 71r is not essential, and that is, a plurality of protrusions 71p, 71p... may be provided in a pattern directly on the circumferential face 71s of the anvil roller 71 or the oscillating surface 61s of the horn 61.

In the foregoing reference example, the ultrasonic-energy regulating unit changes the amount (J/m) of ultrasonic energy based on signals outputted from a rotary encoder, the signals indicating the rotational angle of the rotating drum 30. However, the invention is not limited thereto. For example, the amount (J/m) of ultrasonic energy may be changed as follows: suitable sensors (e.g. a limit switch or a proximity switch) are provided respectively in a front part and a back part of the rotating drum 30 in the CD direction, and the ultrasonic-energy regulating unit detects, based on signals outputted such sensors, whether the anvil roller 71 is in a state immediately before having finished crossing the crossing section A1a, or, whether the anvil roller 71 has just finished the crossing, or whether the anvil roller 71 is in a state immediately after having finished the crossing.

In the foregoing reference example, the amplitude of the ultrasonic vibration for the forward path of reciprocation in the CD direction is larger than the amplitude of the ultrasonic vibration for the return path. However, the invention is not limited thereto. For example, the magnitudes of the amplitudes may be inversed. That is, the amplitude of the ultrasonic vibration for the return path of reciprocation in the CD direction may be larger than the amplitude of the ultrasonic vibration for the forward path.

In the first embodiment, when the horn 61b is passing the crossing section A1a, in both of the forward path and the return path of reciprocation, the oscillating surface 61bs of the horn 61b vibrates ultrasonically in a direction in which the substrate 1a is sandwiched. This can reduce kinetic frictional resistance which may generate between the horn 61b and the substrate 1a when the horn 61b is passing the crossing section A1a. Accordingly, the horn 61b can reciprocate smoothly.

In the foregoing embodiment, as an example of the absorbent article, a disposable diaper 1 is described which is attached to a wearer and absorbs excretion liquid. However, the invention is not limited thereto. That is, anything which absorbs excretion liquid such as urine or menstrual blood may be employed as an absorbent article according to the present invention. For example, a sanitary napkin and a pet pad absorbing excretion liquid of pets are also included in the concept of an absorbent article according to the present invention.

In the foregoing embodiment, rotating member is exemplified by the rotating drum 30. However, the invention is not limited thereto. That is, it is possible to use, without any problem, a member which is capable of rotating along the outer circumferential face 30s while holding the substrate 1a of the absorbent article 1 on the outer circumferential face 30s. For example, an endless belt which is wound around a pair of rollers may be employed as a rotating member.

In the reference example, as shown in FIG. 3, the substrate 1a, which is an example of the sheet-like member 1a, is a continuous body in the transport direction. However, the invention is not limited thereto. For example, the substrate 1a may be a single-cut member having a size corresponding to a single diaper. However, in this case, it is preferable that a mechanism for actively holding the substrate 1a is provided on the outer circumferential face 30s of the rotating drum 30. For example, it is preferable that a plurality of suction holes are formed on the outer circumferential face 30s to attach the substrate 1a onto outer circumferential face 30s by sucking from the suction holes.

In the reference example, as shown in FIG. 6, a driving mechanism that reciprocates in the CD direction the support unit 73 associated with the anvil roller 71 is exemplified by the cam mechanism. The cam mechanism is exemplified by the followings: the ribbed cam 51r provided on the outer circumferential face 51s of the cylindrical member 51; and a pair of cam followers 53 and 53 which are provided in the base section 73b of the support unit 73 and which engage with the cam 51r by sandwiching it therebetween in the front-back direction. However, the invention is not limited thereto. For example, the following configuration is also acceptable: an endless grooved cam is provided based on the foregoing cam curve on the outer circumferential face 51s of the cylindrical member 51; a cam follower is provided on the base section 73b; and the cam follower fits into the grooved cam and engages with it.

In the forward path and the return path of the reciprocation in the CD direction, there are sections Aef and Aeb except for the crossing section A1a. That is, in the forward path and the return path, the sections Aef and Aeb are located closer to the ends in the CD direction than the crossing section A1a is. In other words, in the forward path and the return path, the substrate 1a does not exist in the sections Aef and Aeb. In the reference example, the magnitude (N) of sandwiching force of the horn 61 and the anvil roller 71 in the sections Aef and Aeb is not described in detail. By separating the anvil roller 71 from the horn 61 in the sections Aef and Aeb, the magnitude (N) of pressing force to the horn 61 (the foregoing sandwiching force) may be zero. A method for the separation can be exemplified by the following: when the anvil roller 71 has reached a position close to an end in the CD direction with respect to the crossing section A1a, the anvil roller 71 is moved by a cam mechanism outwards in the rotation-radius direction Dr30 of the rotating drum 30, the cam mechanism being additionally provided in the outer circumferential face 30s of the rotating drum 30. This separation operation makes it possible to effectively prevent the followings: damage or wear of components of the ultrasonic processing apparatus 20 caused by metallic contact of the anvil roller 71 with the horn 61 that vibrates ultrasonically; contamination of the substrate 1a caused by wear metal powder; and the like.

In the foregoing reference example, the CD direction, which intersects the transport direction of the substrate 1a, is exemplified by a direction orthogonal to the transport direction. However, the invention is not limited thereto. That is, the CD direction may be slightly inclined to the direction orthogonal to the transport direction.

### Reference Signs List

1 disposable diaper (absorbent article), 1LH leg opening,
1BH waist opening,
1a substrate (sheet-like member), 1ae edge, 1ap part, 1c part,
1e side-end part (to-be-welded part),
2a continuous sheet, 2a1 continuous sheet, 2a2 continuous sheet,
4 absorbent main body, 6 elastic member, 7 elastic member,
10 front piece, 11 back piece, 13 crotch part,
14 welded part,
20 ultrasonic processing apparatus, 20b ultrasonic welding apparatus,
30 rotating drum (rotating member, cylinder), 30s outer circumferential face, 30SL notch,
30M servomotor (driving source), 30MPL pulley,
30TB timing belt,
31 shaft member, 31Brg bearing member,
31PL pulley, 31eb one end section ,
41 column, 41eb one end section , 41w wall section,
45 linear guide, 45R rail, 45SB sliding block ,
51 cylindrical member, 51s outer circumferential face,
51r ribbed cam, 53 cam follower,
55 support member on ground side,
60 ultrasonic processing unit, 60a ultrasonic processing unit,
60b ultrasonic processing unit, 60c ultrasonic processing unit,
61 horn (rail-like member, ultrasonic processing member), 61s oscillating surface,
61b horn (ultrasonic processing member), 61bs side surface (oscillating surface),
71 anvil roller (anvil, ultrasonic processing member),
71A shaft section, 71APL pulley,
71s circumferential face, 71r rib, 71rs top surface,
71p protrusion, 71ps top surface,
73 support unit, 73a support unit,
73TB1 timing belt, 73TB2 timing belt,
73b base section, 73ba base section,
73bas stay member,
73beb back-end part, 73bebPL pulley,
73bh extending part,
73ss seesaw-like member, 73ssef front-end part, 73sseb back-end part ,
73ssp support shaft,
73sspPL another pulley, 73sspPL2 another pulley,
75 air cylinder, 75c cylinder section, 75pr piston rod,
76 air spring, 76b bag body
90a guide roll, 90b guide roll,
A1a crossing section,
Aef section of path except for crossing section, Aeb section of path except for crossing section,
Pf forward limit , Pb backward limit ,
Rw winding angular range, Rw1 first angular range, Rw2 second angular range,
Rw3 angular range except for first and second angular range,
Pws winding start position, Pwe winding end position,
G space, GND ground,
C30 central axis, C71 rotational axis.

## Claims

1. An ultrasonic welding apparatus (20) that performs an ultrasonic welding process to a sheet-like member (1a),
the sheet-like member being associated with an absorbent article (1),
the sheet-like member being transported while being wound onto an outer circumferential face (30s) of a rotating member (30),
the rotating member rotating about its central axis (C30),
the apparatus comprising:
the rotating member (30);
an ultrasonic processing unit (60)
that rotates about the central axis together with the rotating member, and
that includes as ultrasonic processing members
a horn (61) that vibrates ultrasonically and
an anvil (71) between which and the horn the sheet-like member is sandwiched; and
the horn and the anvil being guided so as to reciprocate in a CD direction,
the CD direction intersecting a transport direction of the sheet-like member,
the horn and the anvil reciprocating in the CD direction with respect to a part (lap) of the sheet-like member,
the part being wound around the rotating member (30), **characterized in that**
both of the horn (61) and the anvil (71) are in contact with the sheet-like member in a predetermined section during movement in both of a forward path and a return path of the reciprocation, with both of the horn (61) and the anvil (71) reciprocating in the CD direction while the sheet-like member (1a) is sandwiched between the horn and the anvil,
the predetermined section being included in the outer circumferential face of the rotating member and extending in the CD direction,
in both of the forward path and the return path, the horn (61) vibrates ultrasonically when the ultrasonic processing member is passing the predetermined section,
the apparatus further comprising an ultrasonic-energy regulating unit,
the ultrasonic-energy regulating unit making
an amount (J/m) of applied ultrasonic energy per unit length in the CD direction of the predetermined section of the forward path
different from
an amount (J/m) of applied ultrasonic energy per unit length in the CD direction of the predetermined section of the return path.

2. An ultrasonic welding apparatus of a sheet-like member associated with an absorbent article according to claim 1, wherein
the ultrasonic-energy regulating unit changes an amount of the ultrasonic energy (J/m) that is applied per unit length, by changing an amplitude of ultrasonic vibration of the horn (61).

3. An ultrasonic welding apparatus of a sheet-like member associated with an absorbent article according to claim 1, wherein
the ultrasonic-energy regulating unit changes an amount of the ultrasonic energy (J/m) that is applied per unit length, by changing a magnitude of sandwiching force at which the sheet-like member (1a) is sandwiched between the horn (61) and the anvil (71).

4. An ultrasonic welding apparatus of a sheet-like member associated with an absorbent article according to claim 1, wherein
the ultrasonic-energy regulating unit changes an amount of the ultrasonic energy (J/m) that is applied per unit length, by changing a size of a space (G) between the horn (61) and the anvil (71).

5. An ultrasonic welding apparatus of a sheet-like member associated with an absorbent article according to claim 1, wherein
the ultrasonic-energy regulating unit changes an amount of the ultrasonic energy (J/m) that is applied per unit length, by changing a travel speed value at which the ultrasonic processing member moves in the CD direction.

6. An ultrasonic welding apparatus of a sheet-like member associated with an absorbent article according to any one of claims 1 to 5, wherein
in at least either one of the horn (61) and the anvil (71), a plurality of protrusions are provided so that welded parts are formed in a pattern in the sheet-like member (1a).

7. An ultrasonic welding method in which a sheet-like member (1a) associated with an absorbent article (1) is subject to an ultrasonic welding process,
the sheet-like member being transported on an outer circumferential face (30s) of a rotating member (30),
the rotating member rotating about its central axis (C30),
the method comprising:
rotating an ultrasonic processing unit (60) about the central axis together with the rotating member,
the ultrasonic processing unit including as ultrasonic processing members
a horn (61) and
an anvil (71) facing the horn;
causing the horn to vibrate ultrasonically;
causing the horn and the anvil to reciprocate in a CD direction with respect to a part (lap) of the sheet-like member, the CD direction intersecting a transport direction of the sheet-like member,
the part being wound around the rotating member (30),
**characterized in that** the horn and the anvil are in contact with the sheet-like member in a predetermined section during movement in both of a forward path and a return path of the reciprocation, the reciprocating being performed while the sheet-like member is sandwiched between the horn and the anvil;
the predetermined section being included in the outer circumferential face of the rotating member and extending in the CD direction,
in both of the forward path and the return path, the horn (61) vibrates ultrasonically when the ultrasonic processing member is passing the predetermined section,
an amount (J/m) of applied ultrasonic energy per unit length in the CD direction of the predetermined section of the forward path being different from
an amount (J/m) of applied ultrasonic energy per unit length in the CD direction of the predetermined section of the return path.

## Patentansprüche

1. Ultraschall-Schweißvorrichtung (20), die einen Ultraschall-Schweißprozess an einem flächigen Element (1a) ausführt,
wobei das flächige Element mit einem saugfähigen Artikel (1) assoziiert ist,
wobei das flächige Element transportiert wird, während es auf eine Außenumfangsfläche (30s) eines rotierenden Elements (30) gewickelt ist,
wobei das rotierende Element um seine Mittelachse (C30) rotiert,
wobei die Vorrichtung Folgendes umfasst:
das rotierende Element (30);
eine Ultraschall-Verarbeitungseinheit (60), die zusammen mit dem rotierenden Element um die Mittelachse rotiert, und
die als Ultraschall-Verarbeitungselemente Folgendes umfasst:
ein Horn (61) das mit Ultraschallfrequenz schwingt, und
einen Amboss (71), wobei das flächige Element zwischen diesem und dem Horn geklemmt ist; und
wobei das Horn und der Amboss geführt sind, um sich in einer CD-Richtung hin- und herzubewegen,
wobei die CD-Richtung eine Transportrichtung des flächigen Elements kreuzt,
wobei sich das Horn und der Amboss in Bezug auf einen Teil (10ap) des flächigen Elements in der CD-Richtung hin- und herbewegen,
wobei der Teil um das rotierende Element (30) gewickelt ist,
**dadurch gekennzeichnet, dass**
sich sowohl das Horn (61) als auch der Amboss (71) in einem vorbestimmten Abschnitt während der Bewegung entlang sowohl einer Vorwärtsbahn als auch einer Rückkehrbahn der Hin- und Herbewegung mit dem flächigen Element in Kontakt befinden, wobei sich sowohl das Horn (61) als auch der Amboss (71) in der CD-Richtung hin- und herbewegen, während das flächige Element (1a) zwischen dem Horn und dem Amboss geklemmt ist,
wobei der vorbestimmte Abschnitt in der Außenumfangsfläche des rotierenden Elements enthalten ist und sich in der CD-Richtung erstreckt,
wobei das Horn (61) auf sowohl der Vorwärtsbahn als auch der Rückkehrbahn mit Ultraschallfrequenz schwingt, wenn das Ultraschall-Verarbeitungselement den vorbestimmten Abschnitt passiert,
wobei die Vorrichtung weiter eine Ultraschallenergie-Reguliereinheit umfasst,
wobei die Ultraschallenergie-Reguliereinheit bewirkt:
dass eine Menge (J/m) der pro Längeneinheit in der CD-Richtung des vorbestimmten Abschnitts der Vorwärtsbahn aufgebrachten Ultraschallenergie
verschieden ist von
einer Menge (J/m) der pro Längeneinheit in der CD-Richtung des vorbestimmten Abschnitts der Rückkehrbahn aufgebrachten Ultraschallenergie.

2. Ultraschall-Schweißvorrichtung eines mit einem saugfähigen Artikel assoziierten flächigen Elements nach Anspruch 1, wobei
die Ultraschallenergie-Reguliereinheit eine Menge der Ultraschallenergie (J/m), die pro Längeneinheit aufgebracht wird, ändert, indem sie eine Amplitude der Ultraschallschwingung des Horns (61) ändert.

3. Ultraschall-Schweißvorrichtung eines mit einem saugfähigen Artikel assoziierten flächigen Elements nach Anspruch 1, wobei
die Ultraschallenergie-Reguliereinheit eine Menge der Ultraschallenergie (J/m), die pro Einheitslänge aufgebracht wird, ändert, indem sie einen Betrag der Klemmkraft ändert, mit der das flächige Element (1a) zwischen dem Horn (61) und dem Amboss (71) geklemmt ist.

4. Ultraschallenergie-Schweißvorrichtung eines mit einem saugfähigen Artikel assoziierten flächigen Elements nach Anspruch 1, wobei
die Ultraschallenergie-Reguliereinheit eine Menge der Ultraschallenergie (J/m), die pro Längeneinheit aufgebracht wird, ändert, indem sie eine Größe eines Zwischenraums (G) zwischen dem Horn (61) und dem Amboss (71) ändert.

5. Ultraschallenergie-Schweißvorrichtung eines mit einem saugfähigen Artikel assoziierten flächigen Elements nach Anspruch 1, wobei
die Ultraschall-Reguliereinheit eine Menge der Ultraschallenergie (J/m), die pro Längeneinheit aufgebracht wird, ändert, indem sie einen Verfahrgeschwindigkeitswert, mit dem sich das Ultraschall-Verarbeitungselement in der CD-Richtung bewegt, ändert.

6. Ultraschall-Schweißvorrichtung eines mit einem saugfähigen Artikel assoziierten flächigen Elements nach einem der Ansprüche 1 bis 5, wobei
in dem Horn (61) und/oder dem Amboss (71) eine Vielzahl von Vorsprüngen vorgesehen ist, sodass geschweißte Teile in einem Muster in dem flächigen Element (1a) gebildet werden.

7. Ultraschall-Schweißverfahren, bei dem ein mit einem saugfähigen Artikel (1) assoziiertes flächiges Element (1a) einem Ultraschall-Schweißprozess unterzogen wird,
wobei das flächige Element auf einer Außenumfangsfläche (30s) eines rotierenden Elements (30) transportiert wird,
wobei das rotierende Element um seine Mittelachse (C30) rotiert:
wobei das Verfahren Folgendes umfasst:
Rotieren einer Ultraschall-Verarbeitungseinheit (60) um die Mittelachse zusammen mit dem rotierenden Element,
wobei die Ultraschall-Verarbeitungseinheit als Ultraschall-Verarbeitungselemente Folgendes umfasst:
ein Horn (61), und
einen dem Horn gegenüberliegenden Amboss (71);
Bewirken, dass das Horn mit Ultraschallfrequenz schwingt;
Bewirken, dass sich das Horn und der Amboss in Bezug auf einen Teil (1ap) des flächigen Elements in einer CD-Richtung hin- und herbewegen, wobei die CD-Richtung eine Transportrichtung des flächigen Elements kreuzt,
wobei der Teil um das rotierende Element (30) gewickelt ist,
**dadurch gekennzeichnet, dass** sich das Horn und der Amboss in einem vorbestimmten Abschnitt während der Bewegung auf sowohl einer Vorwärtsbahn als auch einer Rückkehrbahn der Hin- und Herbewegung mit dem flächigen Element in Kontakt befinden, wobei die Hin- und Herbewegung ausgeführt wird, während das flächige Element zwischen dem Horn und dem Amboss geklemmt ist;
wobei der vorbestimmte Abschnitt in der Außenumfangsfläche des rotierenden Elements enthalten ist und sich in der CD-Richtung erstreckt,
wobei auf sowohl der Vorwärtsbahn als auch der Rückkehrbahn das Horn (61) mit Ultraschallfrequenz schwingt, wenn das Ultraschall-Verarbeitungselement den vorbestimmten Abschnitt passiert,
wobei eine Menge (J/m) der pro Längeneinheit in der CD-Richtung des vorbestimmten Abschnitts der Vorwärtsbahn aufgebrachten Ultraschallenergie von einer Menge (J/m) der pro Längeneinheit in der CD-Richtung des vorbestimmten Abschnitts der Rückkehrbahn aufgebrachten Ultraschallenergie verschieden ist.

## Revendications

1. Appareil de soudage ultrasonique (20) qui effectue un processus de soudage ultrasonique sur un élément en forme de feuille (la),
cet élément en forme de feuille étant associé à un article absorbant (1),
cet élément en forme de feuille étant transporté tout en étant enroulé sur une face circonférentielle extérieure (30s) d'un élément rotatif (30), cet élément rotatif tournant autour de son axe central (C30),
cet appareil comprenant :
l'élément rotatif (30) ;
une unité de traitement ultrasonique (60)
qui tourne autour de l'axe central avec l'élément rotatif, et
qui comprend comme éléments de traitement ultrasoniques
un pavillon (61) qui vibre ultrasoniquement et
une enclume (71) entre laquelle et le pavillon l'élément en forme de feuille est pris en sandwich ; et
le pavillon et l'enclume étant guidés de façon à effectuer un mouvement de va-et-vient dans une direction CD,
cette direction CD coupant une direction de transport de l'élément en forme de feuille,
le pavillon et l'enclume effectuant un mouvement de va-et-vient dans la direction CD par rapport à une partie (recouvrement) de l'élément en forme de feuille,
cette partie étant enroulée autour de l'élément rotatif (30),
**caractérisé en ce que**
le pavillon (61) et l'enclume (71) sont tous les deux en contact avec l'élément en forme de feuille dans une section prédéterminée pendant le mouvement à la fois dans un trajet vers l'avant et un trajet de retour du mouvement de va-et-vient, avec le pavillon (61) et l'enclume (71) effectuant tous les deux un mouvement de va-et-vient dans la direction CD tandis que l'élément en forme de feuille (la) est pris en sandwich entre le pavillon et l'enclume,
la section prédéterminée étant comprise dans la face circonférentielle extérieure de l'élément rotatif et s'étendant dans la direction CD,
à la fois dans le trajet vers l'avant et dans le trajet de retour, le pavillon (61) vibre ultrasoniquement lorsque l'élément de traitement ultrasonique passe devant la section prédéterminée,
cet appareil comprenant en outre une unité de régulation d'énergie ultrasonique,
cette unité de régulation d'énergie ultrasonique faisant une quantité (J/m) d'énergie ultrasonique appliquée par unité de longueur dans la direction CD de la section prédéterminée du trajet vers l'avant différente d'une quantité (J/m) d'énergie ultrasonique appliquée par unité de longueur dans la direction CD de la section prédéterminée du trajet de retour.

2. Appareil de soudage ultrasonique d'un élément en forme de feuille associé à un article absorbant selon la revendication 1, dans lequel
l'unité de régulation d'énergie ultrasonique change une quantité de l'énergie ultrasonique (J/m) qui est appliquée par unité de longueur, en changeant une amplitude de vibration ultrasonique du pavillon (61).

3. Appareil de soudage ultrasonique d'un élément en forme de feuille associé à un article absorbant selon la revendication 1, dans lequel
l'unité de régulation d'énergie ultrasonique change une quantité de l'énergie ultrasonique (J/m) qui est appliquée par unité de longueur, en changeant une grandeur de la force de prise en sandwich à laquelle l'élément en forme de feuille (la) est pris en sandwich entre le le pavillon (61) et l'enclume (71).

4. Appareil de soudage ultrasonique d'un élément en forme de feuille associé à un article absorbant selon la revendication 1, dans lequel
l'unité de régulation d'énergie ultrasonique change une quantité de l'énergie ultrasonique (J/m) qui est appliquée par unité de longueur, en changeant une taille d'un espace (G) entre le pavillon (61) et l'enclume (71).

5. Appareil de soudage ultrasonique d'un élément en forme de feuille associé à un article absorbant selon la revendication 1, dans lequel
l'unité de régulation d'énergie ultrasonique change une quantité de l'énergie ultrasonique (J/m) qui est appliquée par unité de longueur, en changeant une valeur de vitesse de déplacement à laquelle l'élément de traitement ultrasonique bouge dans la direction CD.

6. Appareil de soudage ultrasonique d'un élément en forme de feuille associé à un article absorbant selon l'une quelconque des revendications 1 à 5, dans lequel
dans au moins soit le pavillon (61), soit l'enclume (71), une pluralité de saillies sont prévues de manière à ce que des parties soudées soient formées selon un motif dans l'élément en forme de feuille (la).

7. Procédé de soudage ultrasonique dans lequel un élément en forme de feuille (la) associé à un article absorbant (1) est soumis à un processus de soudage ultrasonique,
cet élément en forme de feuille étant transporté sur une face circonférentielle extérieure (30s) d'un élément rotatif (30),
cet élément rotatif tournant autour de son axe central (C30),
ce procédé comprenant :
la rotation d'une unité de traitement ultrasonique (60) autour de l'axe central avec l'élément rotatif,
cette unité de traitement ultrasonique comprenant comme éléments de traitement ultrasoniques
un pavillon (61) et
une enclume (71) faisant face au pavillon ;
faisant en sorte que le pavillon vibre ultrasoniquement ;
faisant en sortie que le pavillon et l'enclume effectuent un mouvement de va-et-vient dans une direction CD par rapport à une partie (recouvrement) de l'élément en forme de feuille, la direction CD coupant une direction de transport de l'élément en forme de feuille,
cette partie étant enroulée autour de l'élément rotatif (30),
**caractérisé en ce que** le pavillon et l'enclume sont en contact avec l'élément en forme de feuille dans une section prédéterminée pendant le mouvement à la fois dans un trajet vers l'avant et dans un trajet de retour du mouvement de va-et-vient, le mouvement de va-et-vient étant effectué tandis que l'élément en forme de feuille est pris en sandwich entre le pavillon et l'enclume ;
la section prédéterminée étant comprise dans la face circonférentielle extérieure de l'élément rotatif et s'étendant dans la direction CD,
à la fois dans le trajet vers l'avant et dans le trajet de retour, le pavillon (61) vibre ultrasoniquement lorsque l'élément de traitement ultrasonique passe devant la section prédéterminée,
une quantité (J/m) d'énergie ultrasonique appliquée par unité de longueur dans la direction CD de la section prédéterminée du trajet vers l'avant étant différente
d'une quantité (J/m) d'énergie ultrasonique appliquée par unité de longueur dans la direction CD de la section prédéterminée du trajet de retour.
